(19) Europäisches Patentamt
European Patent Office
Office européen des brevets

(11) Publication number: **0 431 929 B1**

(12) **EUROPEAN PATENT SPECIFICATION**

(45) Date of publication of patent specification: **01.03.95**

(51) Int. Cl.$^6$: **C09K 19/32**, C07C 69/78, C07C 69/753

(21) Application number: **90313233.0**

(22) Date of filing: **06.12.90**

The file contains technical information submitted after the application was filed and not included in this specification

(54) **Carboxylate compounds, liquid crystal compositions and liquid crystal elements containing said compounds and method of optical modulation using said elements.**

(30) Priority: **07.12.89 JP 318456/89**
**23.02.90 JP 43533/90**
**23.02.90 JP 43534/90**
**25.06.90 JP 166393/90**

(43) Date of publication of application:
**12.06.91 Bulletin 91/24**

(45) Publication of the grant of the patent:
**01.03.95 Bulletin 95/09**

(84) Designated Contracting States:
**AT BE CH DE DK ES FR GB GR IT LI LU NL SE**

(56) References cited:
**EP-A- 0 205 340**
**EP-A- 0 341 922**

(73) Proprietor: **MITSUI PETROCHEMICAL INDUSTRIES, LTD.**
**2-5, Kasumigaseki 3-chome**
**Chiyoda-ku**
**Tokyo 100 (JP)**

(72) Inventor: **Shimizu, Toyoji, Mitsui Petrochem. Ind. Ltd.**
**2-5, Kasumigaseki 3-chome**
**Chiyoda-ku,**
**Tokyo 100 (JP)**
Inventor: **Nishiyama, Shinichi, Mitsui Petrochem. Ind. Ltd.**
**580-32, Aza-Taku 2-gou, Nagaura**
**Sodegaura-cho,**
**Kimitsu-gun,**
**Chiba 299-02 (JP)**
Inventor: **Doi, Nobuyuki, Mitsui Petrochem. Ind. Ltd.**
**2-5, Kasumigaseki 3-chome**
**Chiyoda-ku,**
**Tokyo 100 (JP)**
Inventor: **Miyakoshi, Shoichi, Mitsui Petrochem. Ind. Ltd.**
**580-32, Aza-Taku 2-gou,**
**Nagaura,**
**Sodegaura-cho**
**Kimitsu-gun,**
**Chiba 299-02 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 431 929 B1

Inventor: **Yamanaka, Tooru, Mitsui Petrochem. Ind. Ltd.**
**580-32, Aza-Taku 2-gou,**
**Nagaura,**
**Sodegaura-cho**
**Kimitsu-gun,**
**Chiba 299-02 (JP)**
Inventor: **Taniguchi, Katsuo, Mitsui Petrochem. Ind. Ltd.**
**3 Chigusa-Kaigan**
**Ichihara-shi,**
**Chiba 299-01 (JP)**
Inventor: **Hama, Hideo, Mitsui Petrochem. Ind. Ltd.**
**580-32, Aza-Taku 2-gou,**
**Nagaura,**
**Sodegaura-cho**
**Kimitsu-gun,**
**Chiba 299-02 (JP)**

(74) Representative: **Myerscough, Philip Boyd et al**
**J.A.KEMP & CO.**
**14, South Square**
**Gray's Inn**
**London WC1R 5LX (GB)**

## Description

### FIELD OF THE INVENTION

This invention relates to novel carboxylate (carboxylic acid ester) compounds and liquid crystal compositions containing said compounds.

In another aspect, the invention relates to liquid crystal elements containing the novel carboxylate compounds described above, processes for the preparation thereof and methods of light modulation using said elements.

### BACKGROUND OF THE INVENTION

Display devices relying on use of liquid crystal compounds which are widely used therefor at present are driven by TN (twisted nematic) mode.

When this TN mode is adopted, however, there are involved such problems that in order to change the image being displayed, the driving time is prolonged, because the position of the molecule of the liquid crystal compound in the element must be changed, and also the voltage necessary for changing the position of the molecules of the liquid crystal compound, that is, the power consumption becomes large.

In distinction to switching elements utilizing TN mode or STN mode, the switching elements using ferroelectric liquid crystal compounds are able to function as switching elements only by changing the direction of molecular orientation of said liquid crystal compounds and hence the switching time required for operating the switching elements is markedly shortened. Further, because a value of Ps x E obtained from a spontaneous polarization (Ps) of the ferroelectric liquid crystal compound and intensity of the electric field (E) applied is an effective energy output for changing the direction of molecular orientation of said liquid crystal compound, power consumption required therefor can also be markedly minimized. Such ferroelectric liquid crystal compounds as mentioned above are suitable particularly as display devices for moving picture, because they have two steady states depending upon the direction of electric field applied, that is, bistability and also very favorable switching threshold value characteristics.

When these ferroelectric liquid crystal compounds are intended to use in optical switching elements, they are required to have such characteristics as an operating temperature in the vicinity of ordinary temperature or below, a wide operating temperature zone, a high switching speed and an appropriate switching threshold value voltage. Particularly, of these characteristics, the operating temperature range is especially important when the ferroelectric liquid crystal compounds are used in optical switching elements.

So far as ferroelectric liquid crystal compounds known hitherto are concerned, however, they are generally narrow in operating temperature, and even in ferroelectric liquid crystal compounds having a wide operating temperature range, said operating temperature range is in a high temperature zone excluding room temperature, as disclosed in R.B. Meyer et al., J. de Phys., Vol. 36 L, p. 69 (1975) and a paper reported by M. Taguchi and T. Harada, "Proceedings of Eleventh Conference on Liquid Crystal", p. 168 (1985). Thus, no ferroelectric liquid crystal compounds which are satisfactory from the standpoint of practical use are available yet.

By the way, there have heretofore been made various proposals for light modulation elements using such ferroelectric liquid crystal compounds as mentioned above.

For example, these light modulation elements may be driven by a method using a liquid crystal cell composed of two transparent substrates being arranged so as to face each other, leaving a gap of about 2 μm between said substrates, said gap being filled with a ferroelectric liquid crystal assuming a chiral smetic phase C.

The ferroelectric crystal has a layer structure in the chiral smectic phase C, and in this layer a major axis of molecule is oriented so that this axis forms a practically definite angle $\theta$ (called a tilt angle). In this state, as shown in Fig. 4, the major axis of liquid crystal molecule 41 gradually turns owing to interaction between the molecules to a different direction and comes to form a helical structure (Fig. 4).

However, when a gap of about 2 μm formed by two glass substrates is filled with a liquid crystal material, the oriented state of the liquid crystal material is influenced by the glass substrates to release its helical structure, and the liquid crystal molecule 51 comes to exhibit two forms of steady state when viewed from above the transparent substrate 50 as shown in Fig. 5. In the steady state as mentioned above, because the major axis of liquid crystal molecule and a dipole perpendicular thereto take the direction opposite to each other in the two forms of steady state, the steady state of the liquid crystal material can be transferred between the above-mentioned two steady states by applying an electric field thereto.

In that case, the amount of transmitted light can be controlled by arranging the above-mentioned liquid crystal cell between two polarizing plates wherein the directions of polarized light cross at right angles so that the cell becomes dark (the amount of transmitted light decreases) when the liquid crystal in the cell takes one form of the two forms of steady state.

In the process as mentioned above, theoretically it is said that the steady state of liquid crystal material present in the cell involves only two forms as aforesaid. Therefore, it is said that when the liquid crystal material in the cell is once brought to the steady state by allocation thereto of an electric field, said liquid crystal material will not be transferred to another form of the steady state even when the electric field applied is eliminated therefrom, and accordingly the light modulation element comprising the above-mentioned liquid crystal cell comes to have a memory effect.

Actually, however, when the liquid crystal material held in a steady state is allowed to stand, as it is, without application thereto of an electric field, parts of the liquid crystal material are transferred sometimes to another form of steady state, and it is difficult to impart a sufficient memory effect to the light modulation element, that is, it is difficult to maintain the liquid crystal material in a definite steady state at its steady state for a long period of time with application thereto of an electric field. Therefore, in order to maintain the steady state of liquid crystal material, that is, a bright state and a dark state of the light modulation element, it is necessary to apply an electric field thereto to a certain degree.

In the conventional process as mentioned above, the application of an electric field is necessary for attaining even a dark state, and in most cases it was difficult to attain a dark state having a sufficient darkness. On that account, it has been unsuccessful in obtaining a sufficient brightness ratio of a bright state to a dark state, that is, a sufficient contrast.

## OBJECT OF THE INVENTION

The present invention has been made in view of the prior art as mentioned above, and an object of the invention is to provide novel carboxylate compounds usable as liquid crystal compounds capable of forming display devices having such excellent characteristics as broad operating temperature range, high switching speed, appropriate switching threshold value voltage, operability with very small power consumption and high contrast.

Another object of the invention is to provide liquid crystal compositions containing the above-mentioned novel carboxylate compounds and having excellent characteristics.

A still further object of the invention is to provide novel liquid crystal elements having excellent characteristics using the above-mentioned novel carboxylate compounds, processes for the preparation thereof, and methods of light modulation using said elements.

## SUMMARY OF THE INVENTION

The novel carboxylate compounds according to the present invention are represented by the following formula [A].

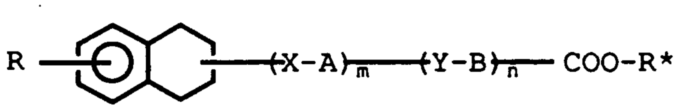

... [A]

wherein R is a group selected from the group consisting of alkyl of 3-20 carbon atoms, alkoxy of 3-20 carbon atoms and halogenated alkyl of 3-20 carbon atoms, X and Y each represent a group selected from the group consisting of -COO-, -OCO-, $-CH_2CH_2-$, $-CH_2O-$, $OCH_2-$, -S-S-,

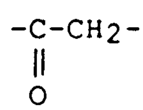

and

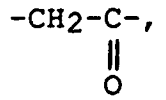

or a single bond, A and B each represents a group selected from the group consisting of

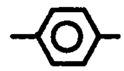 , 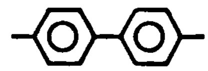 ,

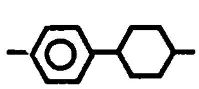 , 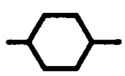 , 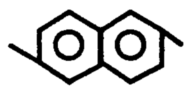 , 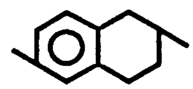 , 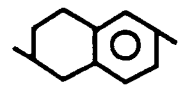 ,

and

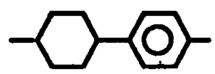

and R* is an optically active group of 4-20 carbon atoms containing at least one asymmetric carbon atom (hydrogen atoms attached to the carbon atoms of said optically active group may be substituted with halogen atoms), and m and n are each an integer of 0-2, with the proviso that both m and n do not become 0 at the same time.

Such novel carboxylate compounds as illustrated above may be used as liquid crystal compounds.

The liquid crystal compositions according to the invention contain the above-mentioned carboxylate compounds.

The first liquid crystal element according to the invention comprises a cell composed of two substrates being arranged so as to face each other leaving a gap between said substrates, said gap being filled with a liquid crystal material which is a liquid crystal composition containing at least one of the above-mentioned carboxylate compounds.

The second liquid crystal element according to the invention comprises a cell composed of two substrates being arranged so as to face each other leaving a gap therebetween, and said gap being filled with a liquid crystal material, wherein each substrate is provided with a transparent electrode on the inner surface thereof, a polarizing plate is provided on the outside of each substrate so that a plane of polarization formed by the polarizing plates has an angle of 70°-110°, and the cell filled with the liquid crystal material is arranged between the polarizing plates at an angle of from +10° to -10°C relative to the position of the cell at which the transmitted light becomes the darkest or the brightest, said liquid crystal material containing the liquid crystal compound represented by the above-mentioned formula [A].

The method of light modulation according to the invention comprises applying an electric field to the above-mentioned second liquid crystal element.

The process according to the invention for the preparation of a liquid crystal element comprising a cell composed of two substrates being arranged so as to face each other leaving a gap therebetween, said gap being filled with a liquid crystal material, which process comprises forming the cell by providing an orientation controlling film on the inner surface of at least one substrate, filling the gap with the liquid crystal material containing the carboxylate compound represented by the above-mentioned formula [A], and heating said liquid crystal material contained in the cell to a temperature not lower than the temperature at which said material exhibits an isotropic liquid, followed by cooling to a temperature not higher than the temperature at which said material exhibits a liquid crystal.

By virtue of using the above-mentioned carboxylate compounds as liquid crystal compounds, there can be obtained various display devices having such excellent characteristics as broad operating temperature range, high switching speed, very small power consumption and stable contrast.

## BRIEF DESCRIPTION OF THE DRAWINGS

Fig. 1 is a chart showing $^1$H-NMR spectrum of R-1'''-trifluoromethylheptyl ester of 4-[4'-(1'',2'',3'',4''-tetrahydro-6''-n-decyloxy-2''-naphthoyloxy) benzoyloxy]benzoic acid.

Fig. 2 is a chart showing $^1$H-NMR spectrum of R-1''-trifluoromethylheptyl ester of 4-(1',2',3',4'-tetrahydro-6'-n-decyloxy-2'-naphthoyloxy)-benzoic acid.

Fig. 3 is a chart showing $^1$H-NMR spectrum of R-1'''-trifluoromethylheptyl ester of 4-[4'-(1'',2'',3'',4''-tetrahydro-6''-n-heptyloxy-2''-naphthoyloxy)benzoyloxy]benzoic acid.

Fig. 4 is a sketch illustrating that each major axis of the molecule of ferroelectric liquid crystal tilts by a tilt angle to the vertical direction of a smetic layer, and the direction of the tilt rotates every smetic layer by a definite angle one by one, thereby assuming a helical structure.

Fig. 5 is a sketch showing the state of a conventional film element wherein two types of the direction of orientation can be attained.

Figs. 6(A) and 6(B) are each a sectional view showing schematically one embodiment of the liquid crystal elements of the present invention.

Figs. 7 and 8 are each a photograph of oscillographic wave showing the relation between a voltage applied at the time when a triangular wave voltage is applied to the liquid crystal element of the present invention in which a liquid crystal cell is arranged so as to obtain the darkest state and an intensity of transmitted light.

Figs. 9 and 10 are each photograph of oscillographic wave showing the relation between a voltage applied at the time when a triangular wave voltage is applied to she liquid crystal element of the present invention in which a liquid crystal cell is arranged so as to obtain the brightest state and an intensity of transmitted light.

## DETAILED DESCRIPTION OF THE INVENTION

The carboxylate compounds according to the present invention, liquid crystal compositions and liquid crystal elements containing the same, and methods of light modulation using said elements are illustrated below in detail.

First, the novel carboxylate compounds of the invention are illustrated.

The novel carboxylate compounds and liquid crystal compounds according to the invention may be represented by the following formula [A].

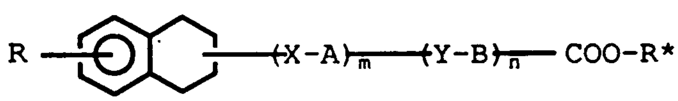

... [A]

In the formula [A], R represents a group selected from the group consisting of alkyl of 3-20 carbon atoms, alkoxy of 3-20 carbon atoms, and halogenated alkyl of 3-20 carbon atoms.

In the above formula [A], when R is alkyl of 3-20 carbon atoms, the alkyl may be any of straight-chain, branched and alicyclic ones. Of the carboxylate compounds of the formula [A], those in which R is straight-chain alkyl, however, exhibit excellent liquid crystal properties, because their molecules assume a rigid structure extending in a straight line. Examples of such straight-chain alkyl includes hexyl, heptyl, octyl, nonyl, decyl, undecyl, dodecyl, tetradecyl, hexadecyl and octadecyl.

When R is halogenated alkyl in the formula [A], examples of such halogenated alkyl include the above-mentioned straight-chain alkyl in which at least a part of the hydrogen atoms has been substituted with a halogen atom such as F, Cl, Br or I.

When R is alkoxy in the above-mentioned formula [A], examples of such alkoxyl include alkoxy having the above-mentioned straight-chain alkyl, for example, hexoxy, heptoxy, octyloxy, nonyloxyl, tetradecyloxy, heptadecyloxy, hexadecyloxy and octadecyloxy, undecyloxyl.

Of the compounds of the above formula [A], those in which R is alkoxyl exhibit particularly excellent liquid crystal properties.

In the formula [A] mentioned above, X and Y each represents a group selected from among -COO-, -OCO-, $-CH_2CH_2-$, $-CH_2O-$, $OCH_2-$, -S-S-,

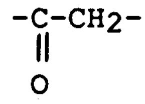

and

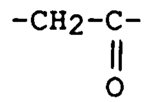,

or a single bond. In the case of the carboxylate compounds of the above formula [A] of the invention used as liquid crystal compounds, X and Y are each selected desirably from among -COO-, -OCO-, $-CH_2CH_2-$, $-CH_2O-$, and $OCH_2-$, and particularly when linearity of the molecule is taken into account, at least one of X and Y is -COO-, preferably both X and Y are -COO-.

In the above-mentioned formula [A], A and B each represent a group selected from the group consisting of

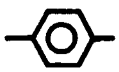, 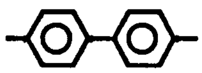, 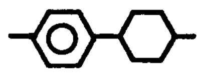, 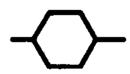, 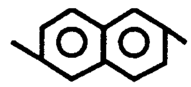, 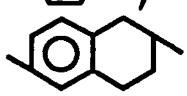, 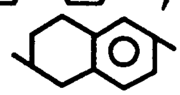,

and

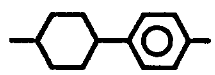.

In the case of the carboxylate compound of the above formula [A] of the invention are used as liquid crystal compounds, when characteristics of liquid crystal material are taken into account, A and B are each preferably the group

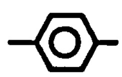, 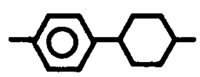,

or

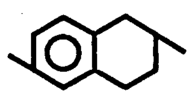.

Of these groups, particularly preferred is phenylene.

In the above formula [A], $R^*$ represents an optically active group of 4-20 carbon atoms having at least one asymmetric carbon atom. The hydrogen atoms attached to the carbon atoms constituting this optically active group may be substituted with halogen atom such as F, Cl, Br or I, particularly with fluorine atom. Examples of such optically active group include those as mentioned below.

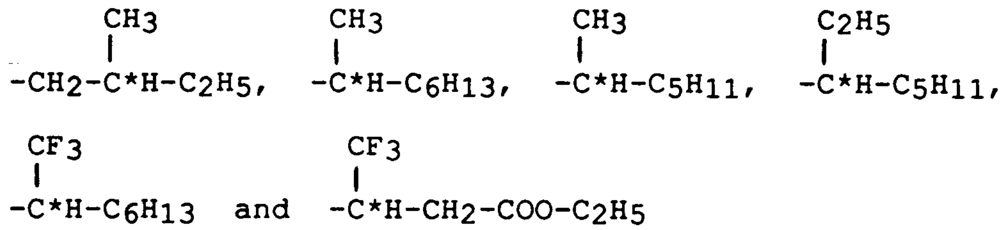

and

Of the optically active groups as mentioned above, preferred are those including

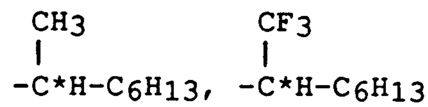

and

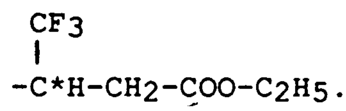

Of these groups, particularly preferred is

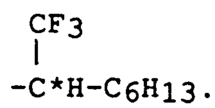

In the formula [A], m and n each represent an integer of 0-2, with the proviso that both m and n do not become 0 at the same time.

In particular, when the carboxylate compounds of the formula [A] are used as liquid crystal compounds, m is preferably 1 or 2.

In the formula [A] mentioned above, 1,2,3,4-tetrahydronaphthyl group includes 1,2,3,4-tetrahydro-1,5-naphthyl, 1,2,3,4-tetrahydro-1,6-naphthyl, 1,2,3,4-tetrahydro-2,6-naphthyl and 1,2,3,4-tetrahydro-1,7-naphthyl.

Particularly, when the carboxylate compounds of the invention are used as liquid crystal compounds, it is preferable that the whole molecule be linear, and particularly preferred as 1,2,3,4-tetrahydronaphthyl group is 1,2,3,4-tetrahydro-2,6-naphthyl.

Accordingly, the carboxylate compounds represented by the above-mentioned formula [A] may include concretely those represented by the formulas [1] to [32].

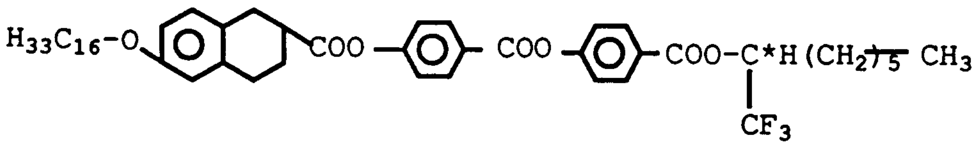 ...[1]

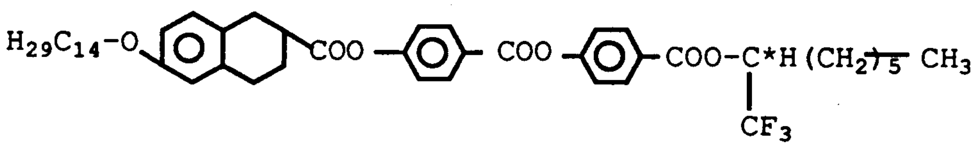 ...[2]

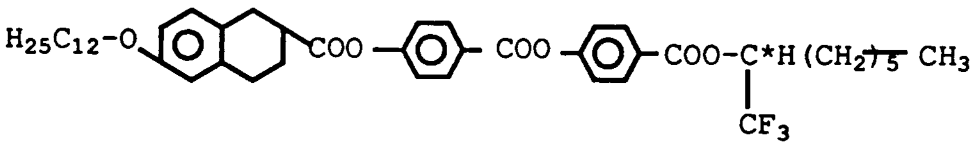 ...[3]

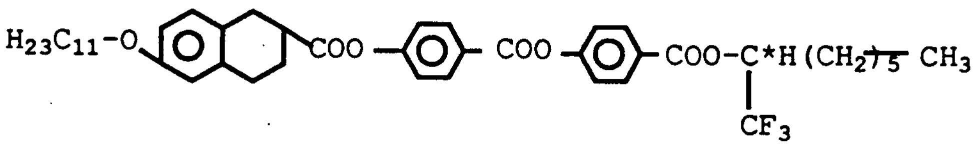 ...[4]

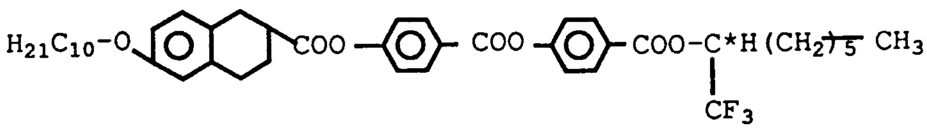

...[5]

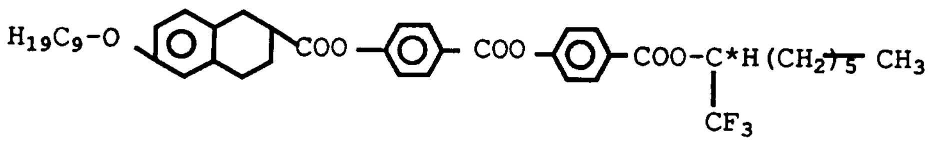

...[6]

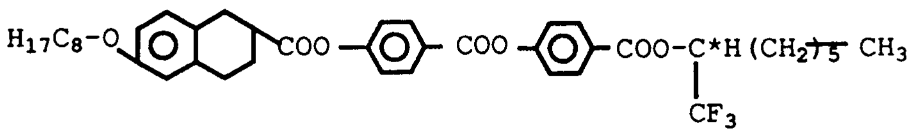

...[7]

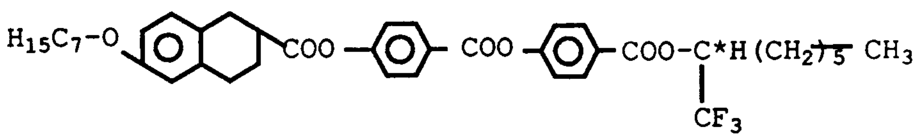

...[8]

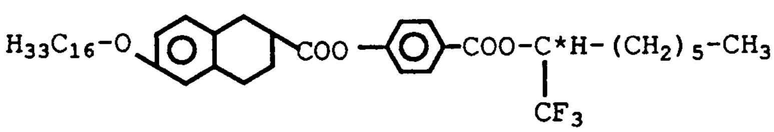

...[9]

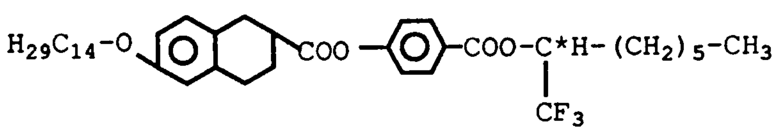

...[10]

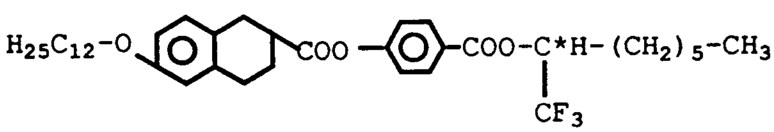

...[11]

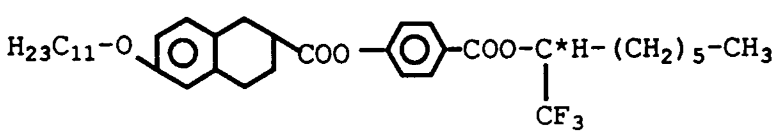

...[12]

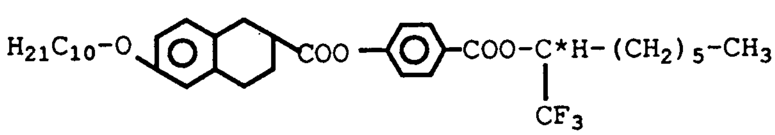

...[13]

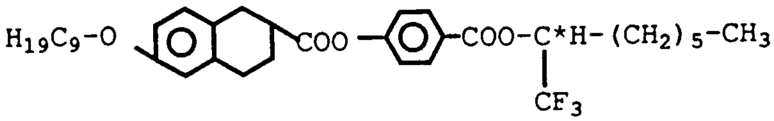

...[14]

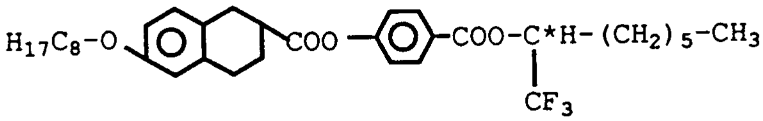

...[15]

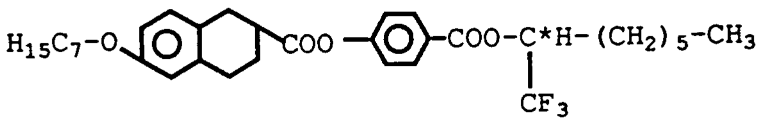

...[16]

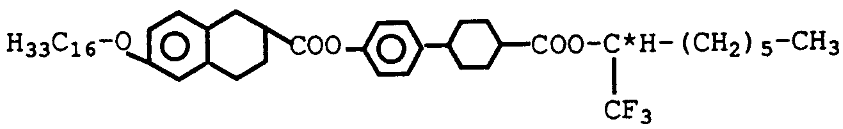

...[17]

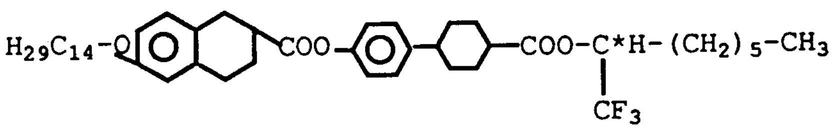

...[18]

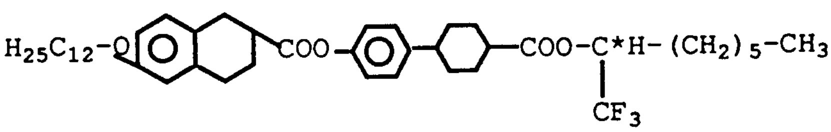

...[19]

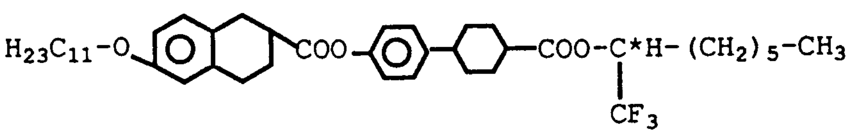

...[20]

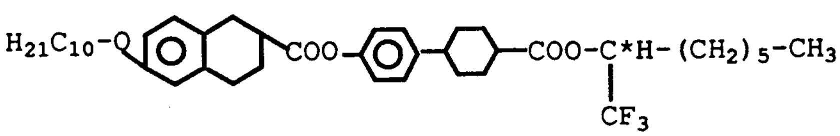

...[21]

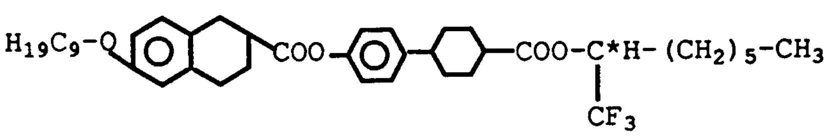

...[22]

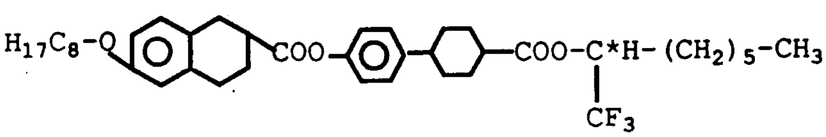

...[23]

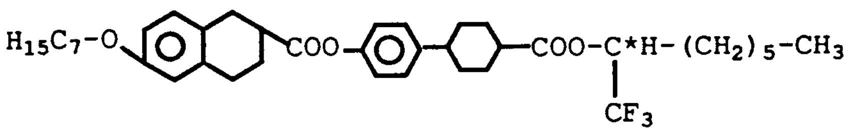

...[24]

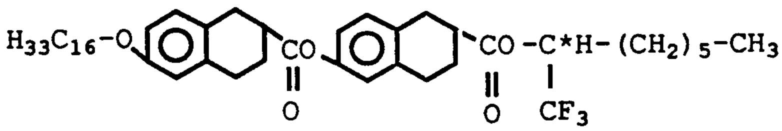

...[25]

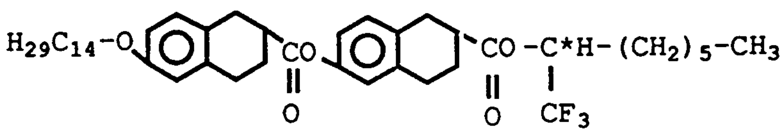

...[26]

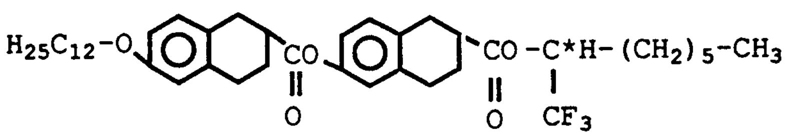

...[27]

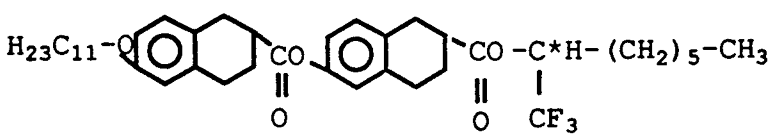

...[28]

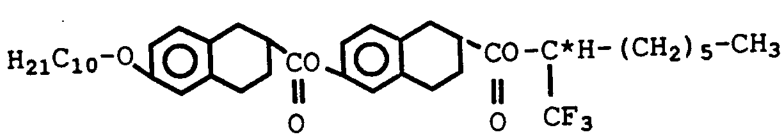

...[29]

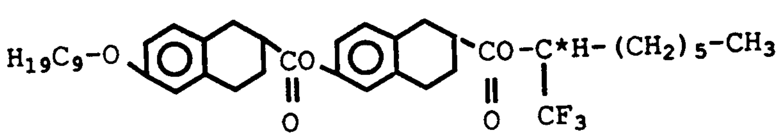

...[30]

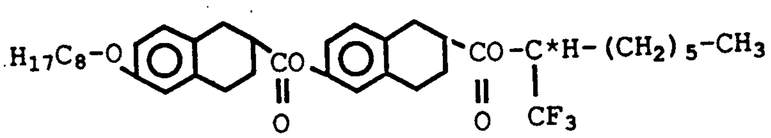

...[31]

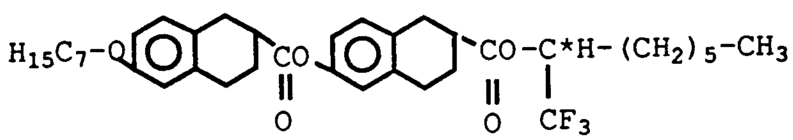

...[32]

The carboxylate compounds as exemplified above may be prepared by utilizing a combination of known synthesis techniques.

For example, the above-exemplified carboxylate compounds may be synthesized through such synthesis route as illustrated below.

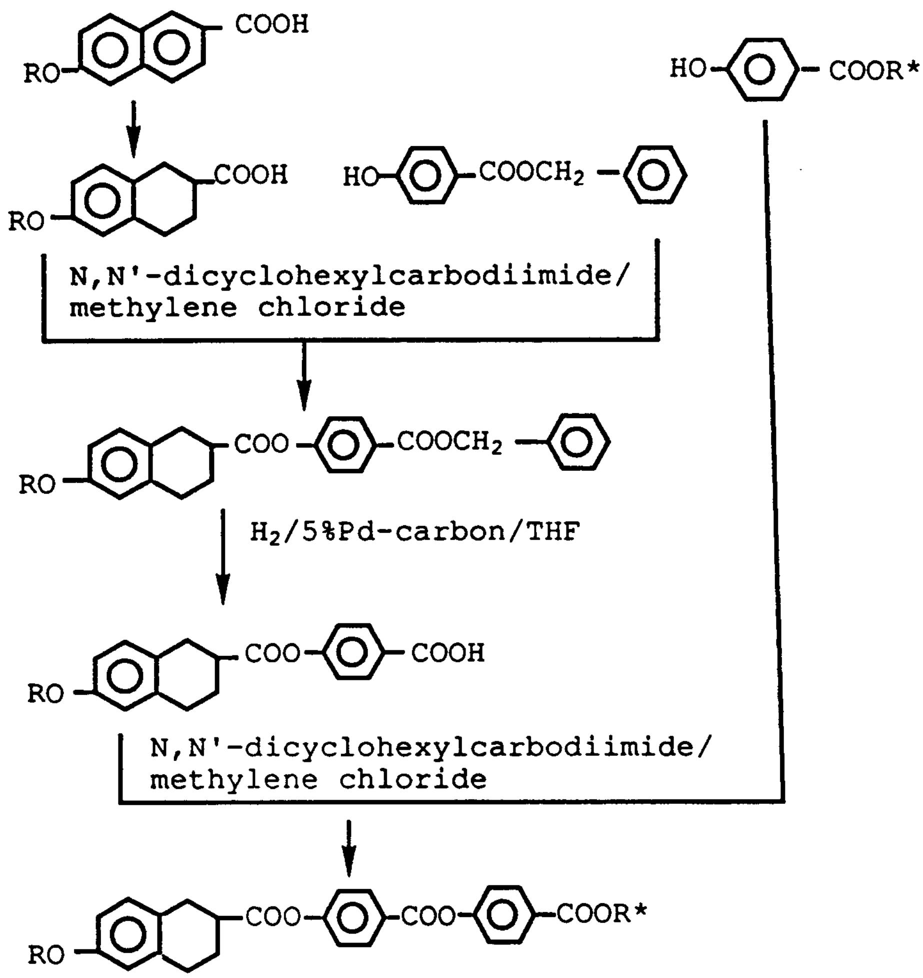


That is, 1,2,3,4-tetrahydro-6-n-alkoxynaphthalene-2-carboxylic acid is obtained, for example, by refluxing a mixture of 6-n-alkoxynaphthalene-2-carboxylic acid and 1,2-ethoxyethane in the presence of metallic sodium while adding dropwise isoamyl alcohol.

The thus obtained 1,2,3,4-tetrahydro-6-n-alkoxynaphthalene-2-carboxylic acid is allowed to react with 4-hydroxybenzoic acid benzyl ester using 4-N,N-dimethylaminopyridine and methylene chloride as solvent while adding dropwise a solution of N,N'-dicyclohexylcarbodiimide to obtain 4-(1',2',3',4'-tetrahydro-6'-n-alkoxy-2'-naphthoyloxy)benzoic acid benzyl ester.

The thus obtained 4-(1',2',3',4'-tetrahydro-6'-n-alkoxy-2'-naphthoyloxy)benzoic acid benzyl ester is poured in a solvent such as tetrahydrofuran, and reduced with hydrogen gas in the presence of a reduction catalyst such as a catalyst composed of palladium on carbon to obtain 4-(1',2',3',4'-tetrahydro-6'-n-alkoxy-2'-naphthoyloxy)benzoic acid.

Subsequently, an ester compound obtained from hydroxybenzoic acid and alcohol having an asymmetric carbon atom is allowed to react with 4-(1',2',3',4'-tetrahydro-6'-n-alkoxy-2'-naphthoxyloxy)benzoic acid obtained in the above step using 4-N,N-dimethylaminopyridine and methylene chloride as a solvent while adding dropwise a solution of N,N'-dicyclohexylcarbodiimide to obtain the carboxylate compound of the present invention.

The process as mentioned above is given as an example of the processes for preparing the carboxylate compounds of the invention, and it should be construed that the carboxylate compounds of the invention are not limited to the above-mentioned process.

Shown in Fig. 1 is a $^1$H-NMR spectrum chart of R-1'''-trifluoromethylheptyl ester of 4-[4'-(1'',2'',3'',4''-tetrahydro-6''-n-decyloxy-2''-naphthoyloxy)benzoyloxy] benzoic acid of the following formula selected, for example, out of the carboxylate compounds of the invention prepared by the above-mentioned process.

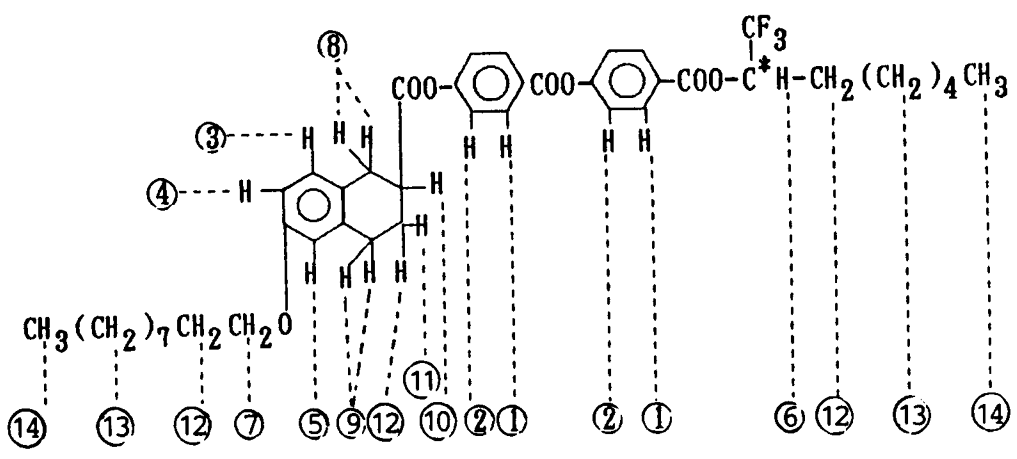

In the above-mentioned formula, numerals 1 to 14 indicate the numbers of hydrogen atoms, and the numbers correspond to numbers attached to the peaks shown in Fig. 1.

Shown in Fig. 2 is a $^1$H-NMR spectrum chart of R-1''-trifluoromethylheptyl ester of 4-(1',2',3',4'-tetrahydro-6'-n-decyloxy-2'-naphthoyloxy)benzoic acid of the following formula selected out of the carboxylate compounds of the invention prepared by the above-mentioned process.

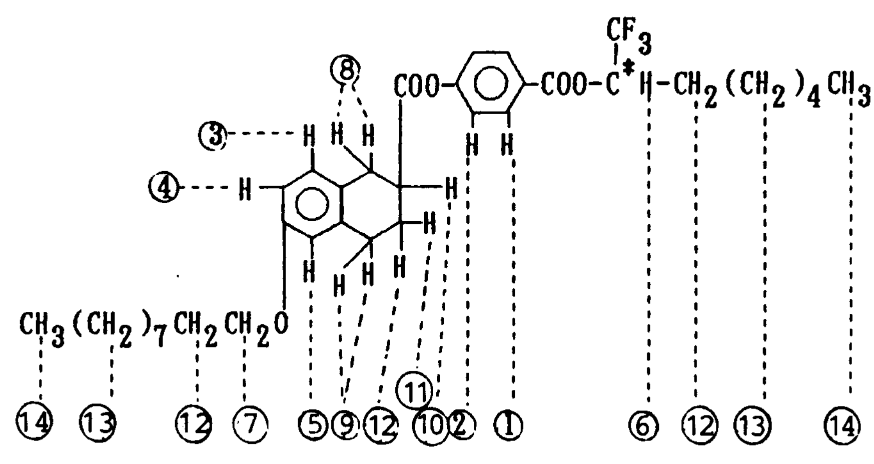

In the above-mentioned formula, numerals 1 to 14 indicate the numbers of hydrogen atoms, and the numbers crrespond to numbers attached to the peaks shown in Fig. 2.

Shown in Fig. 3 is a $^1$H-NMR spectrum chart of R-1'''-trifluoromethylheptyl ester of 4-[4'-(1'',2'',3'',4''-tetrahydro-6''-n-heptyloxy-2''-naphthoyloxy)benzoyloxy]benzoic acid of the following formula selected out of the carboxylate compounds of the invention prepared by the above-mentioned process.

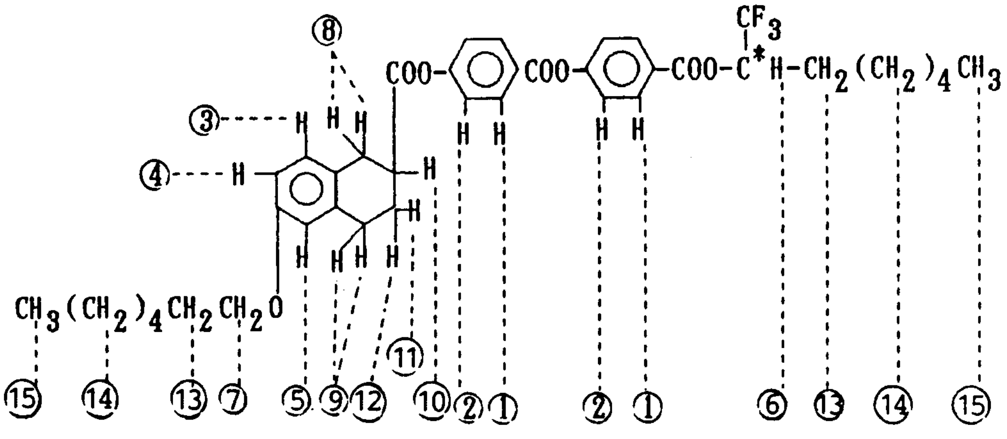

In the above-mentioned formula, numerals 1 to 15 indicate the number of hydrogen atoms, and the numbers correspond to numbers attached to the peaks shown in Fig. 3.

The carboxylate compounds of the formula [A] obtained in the manner now described may be used, for example, as liquid crystal compounds.

Particularly, the carboxylate compounds having optical activity may be used as ferroelectric or anti-ferroelectric liquid crystal compounds.

Of such carboxylate compounds as mentioned above, those represented by the following formulas [5], [8] and [13] exhibit particularly excellent liquid crystal properties.

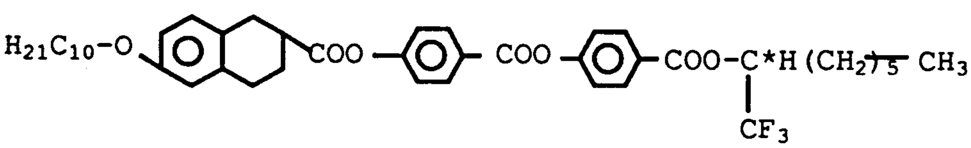 ... [5]

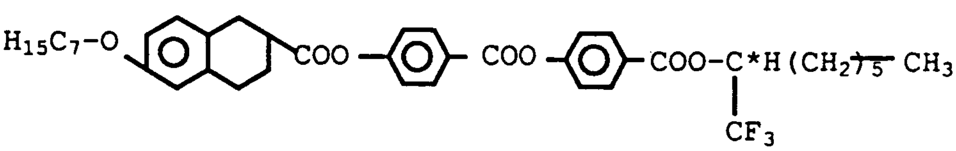 ... [8]

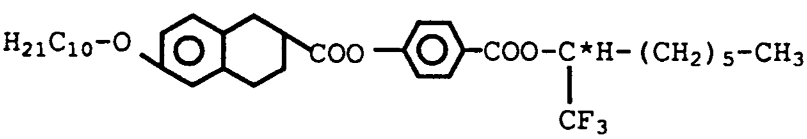 ... [13]

The phase transition temperatures of the compounds represented respectively by the formulas [5], [8] and [13] which are particularly excellent as liquid crystal compounds are shown in Table 1. In the tables that follow, Cry represents a crystal phase, SmC represents a chiral smectic phase, SmA represents a smectic A phase, and Iso represents an isotropic liquid.

Table 1

| Compound | Phase transition temperature | | |
|---|---|---|---|
| | Cry-SmC or SmA | SmC-SmA | SmA-Iso |
| [1] | 30 °C | | 72 °C |
| [5] | 44 °C | 78 °C | 94 °C |
| [8] | <-30 °C | 47 °C | 105 °C |
| [13] | -14 °C | | |

In the liquid crystal compounds of the invention, many compounds assume smectic phase over a wide temperature range as shown in Table 1.

When conventional liquid crystal compounds are used singly, there are scarcely known liquid crystal compounds assuming smectic phase over a wide temperature range as wide as 100 °C as in the case of the above-mentioned compounds of the invention.

The liquid crystal compounds of the invention assume smectic phase over a wide temperature range, and optical-switching elements prepared using such liquid crystal compounds are excellent in high-speed response.

The liquid crystal compounds of the invention may be used singly, and they may also be used as liquid crystal compositions in admixture with other liquid crystal compounds. For example, the liquid crystal compounds of the invention may be used as a principal ingredient in a chiral smectic liquid crystal composition or the compounds may also be used as a minor ingredient in a liquid crystal composition containing other compound assuming smectic phase as a principal ingredient.

In either case, at least one of the above-mentioned liquid crystal compounds of the formula [A] is contained in the liquid crystal compositions of the invention.

In the liquid crystal compositions of the invention, the content of the liquid crystal compound of the formula [A] may be 1-99 parts by weight, preferably 5-75 parts by weight based on the total amount of the liquid crystal materials present in the composition, taking the characteristics, of the liquid crystal compound used, the viscosity, operating temperature and application of the composition into consideration.

The liquid crystal compounds used in the invention, either one or two or more, may be incorporated into the liquid crystal composition.

In liquid crystal compounds which exhibit ferroelectricity like the carboxylate compounds used in the invention, an optical switching phenomenon is induced by application of voltage thereto and, therefore, display devices having good response may be prepared therefrom by utilizing this phenomenon.

The ferroelectric liquid crystal compounds used in such display devices as mentioned above are those assuming any one of a chiral smectic C phase, a chiral smectic F phase, a chiral smectic G phase, a chiral smectic H phase, a chiral smectic I phase, a chiral smectic J phase and a chiral smectic K phase. However, display devices using these liquid crystal compounds are generally slow in response speed except the compounds assuming a chiral smectic C phase ($SmC^*$ phase). Accordingly, driving with a chiral smectic C phase having a quick response speed has heretofore been considered to be advantageous in practical use.

However, the ferroelectric liquid crystal compositions may be used not only in the chiral smectic C phase but also in the smectic A phase by utilizing a method of driving display devices in the smectic A phase as proposed previously by the present inventors (Japanese Patent Application No. 157808/1987).

Because the liquid crystal compounds of the invention exhibit more than two steady states even in liquid phase such as a chiral smectic F phase having a degree of order higher than that of a chiral smectic C phase, they are capable of performing optical switching in the same manner as in the case of a smectic A phase. Accordingly, by using the liquid crystal compositions of the invention containing such carboxylate compounds as mentioned above, there may be obtained liquid crystal elements having a wider liquid crystal temperature range and high-speed electric-optical correspondence.

Table 2 mentioned below shows a case wherein a liquid crystal composition comes to have a wider phase transition temperature range by using such carboxylate compound as mentioned above in the composition. As shown in Table 2, the liquid crystal composition comes to have a wider phase transition temperature range by using R-1'''-trifluoromethylheptyl ester of 4-[4'-(1'',2'',3'',4''-tetrahydro-6''-decyloxy-2''-naphthoyloxy)benzoyloxy]benzoic acid the composition in combination with a liquid crystal material represented by the following formula (B).

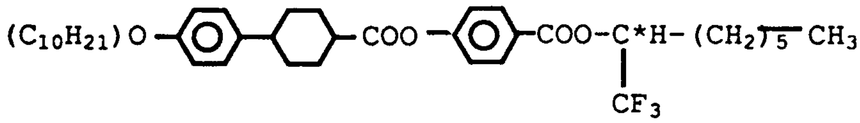 ...[B]

Concretely speaking, the phase transition temperature of from the smectic phase to liquid rises from 53°C to 72°C while maintaining the transition temperature of Cry-SmC* at -30°C.

Table 2

| Compound or composition | Phase transition temperature | | |
|---|---|---|---|
| | Cry-SmC* | SmC*-SmA | SmA-Iso or SmC* |
| [5] | 44 °C | 78 °C | 94 °C |
| [5] 50 wt% + (B) 50 wt% | <-30 °C | 50 °C | 72 °C |
| (B) | 26 °C | | 53 °C |

Note:

In the above Table, [5] represents the compound represented by the following formula,

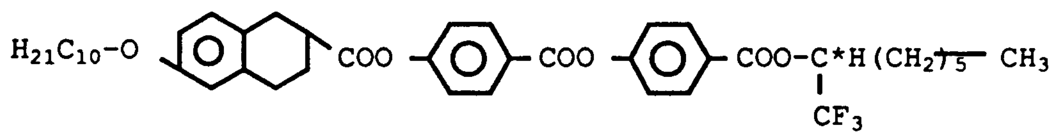

and

(B) represents the compound represented by the following formula.

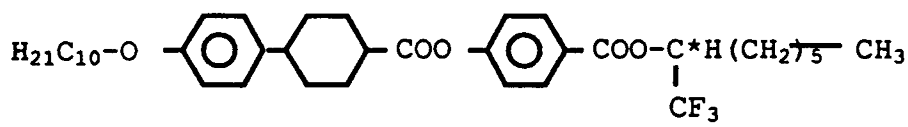

Examples of liquid crystal compounds which can be used together with the liquid crystal compounds of the above-mentioned formula [A] in the liquid crystal compositions of the invention include (+)-4'-(2"-methylbutyloxy)phenyl-6-octyloxynaphthalene-2-carboxylic acid ester, 4'-decyloxyphenyl-6-((+)-2"-methylbutyloxy)naphthalene-2-carboxylic acid ester,

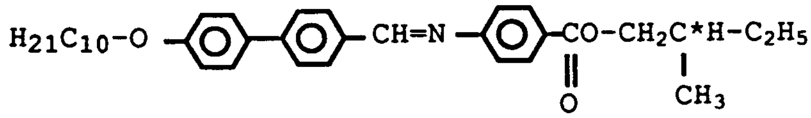

,

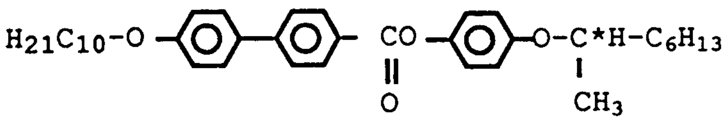

,

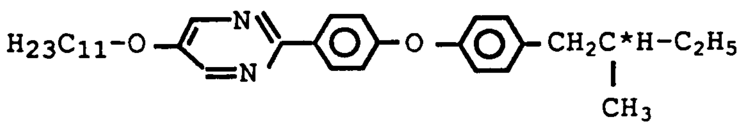

,

Shiff base type liquid crystal compounds such as

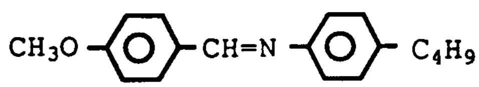

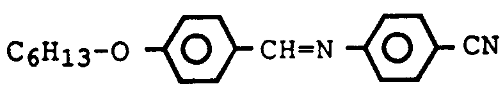

azoxy type liquid crystal compounds such as

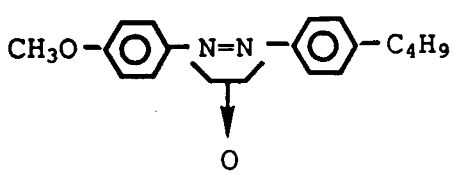

benzoic acid ester type liquid crystal compounds such as

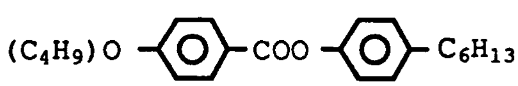

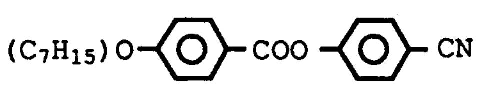

cyclohexylcarboxylic acid ester type liquid crystal compounds such as

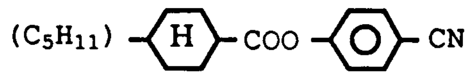

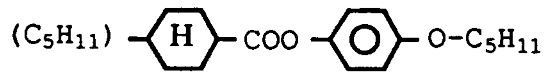

biphenyl type liquid crystal compounds such as

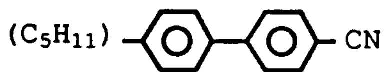

terphenyl type liquid crystal compounds such as

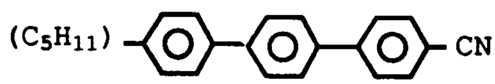

cyclohexyl type liquid crystal compounds such as

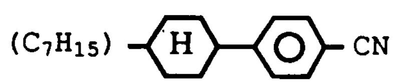

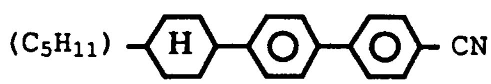

and pyridine type liquid crystal compounds such as

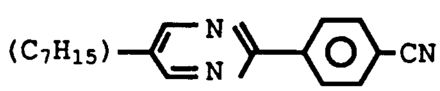

When display elements, for example, those as will be mentioned later, are formed by using the liquid crystal compositions of the invention, additives which can be incorporated into ordinary liquid crystal compositions, for example, conductivity imparting agents and lifetime improving agents, may be added to the present liquid crystal compositions in addition to the above-mentioned carboxylate compounds and other liquid crystal compounds. Further, dichroic dyes can be incorporated into the liquid crystal compositions of the invention when said liquid crystal compositions are used in liquid crystal elements driven by a driving system utilizing the dichroism of dyes.

The liquid crystal compositions of the invention may be prepared by mixing the above-mentioned carboxylate compounds with other liquid crystal compounds and additives if desired.

Liquid crystal element

The liquid crystal elements according to the present invention are now illustrated hereinafter with reference to Figs. 6(A) and 6(B) each showing a section of one embodiment of liquid crystal elements of the invention.

The first liquid crystal element of the invention comprises basically a cell 63 composed of two sheets of transparent substrates 61a and 61b facing each other, and a liquid crystal material 65 charged into a space 64 formed between said substrates 61a and 61b. In other words, the first liquid crystal element of the invention comprises basically a cell 63 composed of the substrates 61a and 61b (hereinafter simply called the substrate) having formed a space 64 therebetween, and a liquid crystal material 65 charged into the space 64 of the cell 63.

In the substrates as mentioned above, at least one of them must be transparent, and usually such a transparent substrate as used herein is made of glass or a transparent plastic such as polycarbonate, 4-methyl-1-pentene polymer or copolymer, amorphous polyolefin such as copolymer of ethylene and tetracyclo[4,4,0,$1^{2.5}$,$1^{7.10}$]3-dodecene.

When a glass substrate is used in the above case, an under coat layer (an unnecessary component permeation preventing layer) comprising such a material, for example, as silicon oxide or the like may also be provided on the inner surface of the glass substrate in order to inhibit deterioration of the liquid crystal material used due to elution of alkali component from said glass substrate.

The transparent substrate, when it is a glass substrate, has a thickness of from 0.01 to 1.2 mm in most cases.

In the present invention, there may also be used transparent flexible substrates as the transparent substrates. In that case, at least one of the transparent substrates may be a flexible transparent substrate or both of them may be flexible transparent substrates.

Useful flexible transparent substrates include, for example, films made of polymer materials.

In these substrates 61a and 61b as illustrated above, usually an electrode 62a and 62b composed of indium-tin oxide are provided on the inner surface thereof, that is, the surface that faces toward the liquid crystal material. In the invention, a transparent electrode substrate composed of a transparent electrode integrally formed on the above mentioned substrate may also be used as the substrate.

The transparent electrode may be formed, for example, by coating indium oxide or tin oxide on the surface of transparent substrate according to the method, per se, known.

The transparent electrode is provided usually to a thickness of from 100 to 2000 Å.

In the liquid crystal element of the invention, it is preferable that an orientation controlling film (orientation layer) is provided on the inner surface of at least one of the two sheets of substrates, particularly on the inner surface of each substrate. Fig. 6(A) shows an embodiment in which two sheets of orientation controlling films 67a and 67b are provided.

The orientation controlling film used in the invention includes organic or inorganic films made of polyimide, silicon oxide, polyvinyl alcohol, polyamide, polyester or the like. Of these films, particularly preferred is a polyimide film.

In preferred embodiments, for example, when one sheet of the orientation controlling film is provided, this one sheet of the orientation controlling film is composed of polyimide, and when two sheets of the orientation controlling film are provided, at least one of them is composed of polyimide, preferably both of them are composed of polyimide.

The polyimide used in that case may be any of polyimides so long as they are polymer materials having imido linkage in the molecule, and such polyimides have preferably those capablity of film forming. Concrete examples of the polyimides include Uprex R (a product of Ube Industries, Ltd.), Sunever 130 (a product of Nissan Chemical Industries, Ltd.), OPTOMER AL1251, JIA-28 (a product of Japan Synthetic Rubber Co., Ltd.), KERMIMID 601 (a product of Nippon Polyimide Co., Ltd.) and HL-1100, LX-1400 (a product of Hitachi Kasei Kogyo K.K.). However, the polyimides used in the invention are not limited to those mentioned above.

As stated above, the polyimides used in the invention are resins consisting essentially of a polymer material having imido linkage. The orientation controlling film used in the invention, however, may contain other resins such as polyamide in addition to the polyimide in such an amount not to have an adverse effect on characteristics of the polyimide, and such resins may be those containing other structural units in addition to imido structural units.

When one of the orientation controlling films is from a material other than the polyimide, this orientation controlling film composed of other material than the polyimide may be composed of an organic or inorganic material.

Examples of the orientation controlling film composed of other material than the polyimide include those composed of such resins, for example, as polyvinyl alcohol, polyamideimide, polyester, polycarbonate, polyvinyl acetal, polyvinyl chloride, polyvinyl acetate, polyamide, polystyrene, siloxane polyimide, cellulose resin, melamine resin, urea resin, acrylic resin and electrically conductive polymer. Further, the orientation controlling film may be a cured article of cyclized rubber photoresist, phenol novolak photoresist or electron beam photoresist such as polymethyl methacrylate or epoxidized 1,4-polybutadiene. Further, the orientation controlling film may be formed from an inorganic material, for example, SiO, $SiO_2$, GeO, $Al_2O_3$, $Y_2O_5$, $ZrO_2$, $MgF_2$ or $CeF_3$.

The orientation controlling film may be formed on the inner surface of each substrate in contact with a liquid crystal by various methods depending on the material used for forming said film, such as a method wherein the above-mentioned resin is applied, for example, by means of spin coating, a method wherein the thus coated resin is heat treated, a method wherein a resin film is laminated, a method wherein a photosensitive resin is applied and then cured by irradiation with an energy ray, and a method wherein an inorganic material is deposited.

Furthermore, the orientation controlling film (orientation layer) may be formed, for example, by chemical adsorption of an organosilane coupling agent or a polynuclear complex of carboxylic acid, or by rhombic deposition of silicon oxide or the like. The orientation layer may also be formed by applying a polyimide resin on the transparent electrode, followed by rubbing the coated polyimide resin in a definite direction.

The orientation layer may be so formed as to serve simultaneously as a spacer as will be mentioned later.

Two sheets of the transparent substrates 61a and 61b as illustrated above are arranged in such a manner that two sheets of the transparent electrodes 62a and 62b are formed on the two sheets of transparent substrates, respectively, so that said two sheets of the transparent electrodes face each other, and that a space into which a liquid crystal material is charged is formed by these two sheets of the substrate.

The width of the space thus formed between the substrates is usually 1 to 10 $\mu$m, preferably 1 to 5 $\mu$m. Such a space as mentioned above may readily be formed, for example, by arranging two sheets of the substrate in position so that the spacer is held between said substrates.

The thickness of the orientation controlling film as illustrated above is in the range of usually 0.005 to 0.25 $\mu$m, preferably 0.01 to 0.15 $\mu$m.

In the present invention, it is desirable that the above-mentioned two sheets of the orientation controlling film are provided respectively on the inner surface of the substrates so that the orientation direction of a liquid crystal material controlled by one of the orientation controlling film and that of the liquid crystal material controlled by the other are nearly parallel to each other, and the orientation directions of said crystal material are in the same direction or in the opposite direction from each other. However, the arrangement of the orientation controlling films as mentioned above is not critical. The orientation controlling films 67a and 67b have the function in orientating the liquid crystal material in the desired direction. Accordingly, the initial orientation of the liquid crystal material is improved and a liquid crystal element excellent in contrast, etc., is obtained by orientating the liquid crystal material by means of the orientation controlling films so arranged that the orientation directions of said liquid material controlled by said orientation controlling films are parallel to each other in the same or opposite direction, as compared with the case wherein the orientation controlling films are arranged is disorderedly.

In the present invention, the orientation controlling film is subjected preferably to orientation treatment. The orientation treatment as referred to herein is intended to designate the treatment for orientating the liquid crystal molecule in the predetermined direction, for example, a polyimide film may be orientated by rubbing said film with cloth or the like in a given direction.

The cell used in the invention comprises two sheets of the transparent substrates 61a and 61b provided, if necessary, with the orientation controlling films 67a and 67b, respectively, in the manner now described, and a space 64 into which the liquid crystal material is charged. The space 64 may be formed, for example, by putting spacers 68 as inner sidewalls between the substrates 61a and 61b. By virtue of providing the spacers 68 in this manner, the space 64 to be filled with the liquid crystal material can be secured and the liquid crystal material charged into the space 64 can be prevented from leaking. The space 64 can be formed by using the above-mentioned spacers capable of forming sidewalls. Alternatively, the space can also be formed by mixing particles (internal spacer) having the predetermined particle diameter with the liquid crystal material.

Useful spacer (internal spacer) as referred to above includes, for example, a polyimide type polymer material obtained by patterning of a photosensitive polyimide precursor. By virtue of using such a spacer as mentioned above, a monodomain is formed by interfacial effect of this spacer with the liquid crystal

material. The orientation film and spacer can also be integrated into one system by using a concentric circular or comb-like spacer which is serviciable as an orientation film.

In addition to the use of the above-mentioned spacers, a given space can be formed between the substrates by mixing fiber with the liquid crystal material so that the substrates form the given space therebetween by the presence of this fiber.

In that case, moreover, the liquid crystal material can be mixed with particles (internal spacer) in place of the fiber, or may be mixed with the fiber together with the particles.

The particles as referred to above include those made of melamine resin, urea resin or benzoguanamine resin having a particle diameter of from 1 to 10 $\mu$m.

The width of the thus formed space between the substrates is usually from 1 to 10 $\mu$m, preferably from 1 to 5 $\mu$m and especially from 1.6 to 5 $\mu$m.

In two sheets of the transparent substrate so arranged as to form a space therebetween by means of a spacer in the manner described above, the peripheries of said substrates are sealed usually with a sealing compound. Such a sealing compound includes epoxy resin, silicone resin, ultraviolet ray curing resin, etc. which may be modified with acrylic material or silicone rubber.

In the liquid crystal element of the invention, various thin films such as a photoconductive film, light screening film, light reflecting film or the like may be provided on the opposite surface of the orientation controlling film formed on the substrate.

In the liquid crystal element, the liquid crystal material 65 is charged into the space 64 of the cell as mentioned above.

The liquid crystal materials used in the invention include carboxylate compounds represented by the aforementioned formula [A]. In the invention, in particular, it is desirable to use liquid crystal compositions containing at least one carboxylate compound of the formula [A], though the carboxylate compound of the formula [A] may also be used singly.

The liquid crystal element of the invention as illustrated above is markedly excellent in contrast, etc., and so may be favorably used as a surface stabilized ferroelectric liquid crystal element, helically strained type element, transient scattering type element, guest-host type element and vertical orientation liquid crystal element.

Using the liquid crystal elements according to the invention, various liquid crystal display devices and electrooptical display devices can be prepared.

Of the liquid crystal elements of the invention, those comprising a cell filled with a liquid crystal composition assuming a smectic phase can be used as memory liquid crystal display devices such as heat writing display element, and laser writing display element. Liquid crystal display devices or electrooptical display devices can be prepared by using such liquid crystal elements, and crystal display devices or electrooptical display devices can be prepared by using such liquid crystal elements.

In addition to the above-mentioned applications, the liquid crystal element of the invention in which a liquid crystal composition containing a carboxylate compound having ferroelectricity is contained can be used as liquid crystal elements such as optical switching elements, e.g., optical shutter and liquid crystal printer, piezoelectric elements and pyroelectric elements, and liquid crystal display devices or electrooptical display devices may be prepared by using such liquid crystal elements.

Namely, when a chiral smectic C phase is formed by using the liquid crystal materials used in the invention, the chiral smectic C phase thus formed exhibits double state stability. Accordingly, when electric field is inverted between bi-stable states, optical switching and display can be performed by using such liquid crystal element as containing a ferroelectric liquid crystal material assuming a chiral smectic C phase.

Further, because such ferroelectric liquid crystal material as assuming a chiral smectic C phase has spontaneous polarization, when voltage is once applied to a cell of liquid crystal element containing said material, the cell will come to have a memory effect even after the electric field is erased. By utilizing this memory effect, therefore, a power consumption of the display device comprising such liquid crystal element can be reduced. In this case, moreover, the contrast of the display device is stabilized and becomes very clear.

The switching element using this chiral smectic liquid crystal compound or composition can be driven at low voltage, because switching can be performed only by changing the direction of molecular orientation of the chiral smectic liquid crystal compound and also because the primary strength of electric field applied to the driving.

When this switching element is used, a high speed response of not more than scores of micro second can be attained. Accordingly, the scanning time of the element can be greatly shortened and a large screen display (liquid crystal display device) having a number of scanning lines can be prepared. Moreover, because this display can be operated at room temperature or lower, scanning can be easily made without

using any auxiliary means for temperature control.

Further, the molecules of the liquid crystal materials used in the liquid crystal elements of the invention are tilted causatively even in the state of a smectic A phase exhibiting no double state stability when an electric field is applied, hence optical switching can be conducted by utilizing this property.

Process for preparing liquid crystal element

A process for preparing the above-mentioned liquid crystal element is illustrated below in detail.

The liquid crystal element of the present invention may be prepared by filling the above-mentioned space between the transparent substrates of the cell with a liquid crystal material including the above-mentioned carboxylate compound.

The liquid crystal material is heated usually to a molten state and then filled (injected) in the above-mentioned space evacuated in advance.

After filling the space with the liquid crystal material, usually the cell is sealed. Subsequently, the liquid crystal material thus filled in the cell is usually subjected to initial orientation. The initial orientation of the liquid crystal material may be performed, for example, by heating the thus sealed cell so that the liquid crystal material present in the cell is heated up to a temperature not lower than the temperature at which said liquid crystal material exhibits an isotropic phase and then cooled to the temperature at which said liquid material exhibit a liquid crystal phase.

In that case, the liquid crystal material is cooled preferably at a rate of not higher than 2°C/min. In particular, this rate of temperature drop employed is preferably in the range of from 0.1 to 2.0°C/min, especially from 0.1 to 0.5°C/min. By cooling the cell at a cooling rate within the range of as defined above, there is obtained a liquid crystal element which is excellent in initial orientation and has a liquid crystal phase. The term initial orientation as used herein refers to the state of arrangement of the liquid crystal material before orientation vector of the liquid crystal material is changed by applying voltage to the liquid crystal element.

Further, the initial orientation of the liquid crystal material thus filled in the space of the liquid crystal cell may be performed, for example, by the temperature gradient method utilizing a spacer edge or the monoaxial orientation controlling method such as surface treatment using an orientation film. In the present invention, moreover, the initial orientation of the liquid crystal material can also be performed by applying an electric field using direct current bias voltage to the liquid crystal material being heated.

The liquid crystal cell thus filled with the liquid crystal material and initially orientated is placed between two polarizing plates, and the two polarizing plates are arranged so that a plane of polarization formed by the polarizing plates has an angle of 70-110°. Preferably, these two polarizing plates are arranged so that the polarization directions of the polarizing plates meet at right angle, that is, the above-mentioned angle becomes 90°.

Useful as the above-mentioned polarizing plates are polarizing films prepared by stretching such resin film, for example, as polyvinyl alcohol resin film or polyvinyl butyrale resin film in the presence of iodine or the like so as to impart polarization to the stretched films. The polarizing film as illustrated above may also be laminated on the surface with other resin so as to have a multi-layer construction.

In the present invention, the liquid cell can be placed between the polarizing plates as arranged in the manner now described, so that the cell is placed in a state to form an angle (rotation angle) within the range of from +10° to -10° (hereinafter abbreviated to ±10°) from the state wherein the amount of transmitted light is the smallest (i.e. the darkest state), preferably in the darkest state. Alternatively, the liquid crystal cell can be placed in a state to form an angle (rotation angle) within the range of ±10° from the state wherein the amount of transmitted light is the largest (i.e. the brightest state), preferably in the brightest state.

Driving (display) method of liquid crystal element

Driving (displaying) the liquid crystal element of the present invention having such structure as mentioned above may be performed, for example, by applying an electric field to said liquid crystal element.

Namely, the liquid crystal element is driven, for example, by applying thereto an electric current of usually 1 Hz-100 KHz, preferably 10 Hz-10 KHz, and an electric field controlled to have a strength of usually 0.01-60 Vp-p/$\mu m^2$, preferably 0.05-30 Vp-p/$\mu m^2$.

When the liquid crystal element is driven by application of an electric field, the amount of light that transmits this element comes to exhibit two kinds of hysteresis curves by changing a wave form (driving

wave) of the electric field applied. That is, the present inventor has been successful in exhibiting memorization in one liquid crystal element by employing two kinds of driving methods. Of the two driving methods, one is to utilize so-called double state stability, and the other is to utilize so-called triple state stability.

A liquid crystal element using MHPOBC as a liquid crystal material is known to exhibit triple state stability, but it exhibits practically no double state stability.

It can be first realized by the liquid crystal element of the present invention that either double state stability or triple state stability can be selected in one kind of liquid crystal element only by operation of changing the wave form (driving wave) of the electric field applied thereto.

Fig. 7 is an oscillograph of an oscillowave form showing the relation between the amount of transmitted light and applied voltage in a liquid crystal element exhibiting triple state stability, and Fig. 8 is also an oscillograph of an oscillowave form showing said relation in a liquid crystal element exhibiting double state stability.

In the liquid crystal element used herein, a liquid crystal cell filled with a liquid crystal material is placed between two polarizing plates so arranged that the planes of polarization thereof meet at right angles, so that the darkest state of the element is attained without applying an electric field thereto. Fig. 7 shows an oscillowave form obtained at the time when a triangular wave of 10 Hz is applied to this liquid crystal element, and Fig. 8 shows an oscillowave form obtained at the time when a triangular wave of 100 Hz is applied to this.

In the liquid crystal element where the liquid crystal cell and polarizing plates are arranged so as to obtain the darkest state in the element, a favorable tri-stable state can be realized by application of an electric field of a relatively low frequency, for example, 0.001-50 Hz, preferably 0.1-30 Hz to the element. The oscillowave form is gradually transformed into the bi-stable state as shown in Fig. 8 with increasing frequency of the electric field applied and a favorable bi-stable state can be realized, for example, by applying an electric field having a frequency of 50 Hz-100 KHz, preferably 70 Hz-10 KHz to the liquid crystal element.

In the liquid crystal element as mentioned above, for example, as shown in Fig. 7, a dark state can be attained when the applied voltage is 0(Vp-p), and in this case the contrast obtained becomes markedly high.

Figs. 9 and 10 respectively show an oscillowave form of a liquid crystal element in which a liquid crystal cell filled with a liquid crystal material is placed between two polarizing plates whose planes of polarization meet at right angle, so that the brightest state of the element is attained. Fig. 9 shows an oscillowave form obtained when a triangular wave of 10 Hz is applied to the liquid crystal element, and Fig. 10 shows an oscillowave form obtained when a triangular wave of 100 Hz is applied to the liquid crystal element. In this liquid crystal element, there is a tendency similar to that in the liquid crystal element used in Figs. 7 and 8, for example, a bi-stable state is attained by applying an electric field having a relatively high frequency.

The electric field applied to the above-mentioned liquid crystal elements is preferably selected from among a rectangular wave (or pulse wave), triangular wave, sinusoidal wave and a combination thereof. When a rectangular wave (or pulse wave or a combination of both) is applied to the liquid crystal element, a rate driving the liquid crystal element can be increased by reducing the width of the applied electric field to not more than 10 millisecond, preferably in the range of from 0.01 to 10 millisecond, and in this region, the liquid crystal element of the invention may be used as a bi-stable state type liquid crystal element. Further, by employing this electric field having the width of larger than 10 millisecond, preferably in the range of from 33 to 1000 millisecond, the liquid crystal element of the invention may be used as a tri-stable state type liquid crystal element in the region where no so high driving is required. The width of an electric field as used herein is intended to designate, for example, in a rectangular wave, a length (i.e. time) of the electric field maintained at a given voltage.

To this liquid crystal element, an electric field can be applied while varying it between negative voltage and positive voltage through OV. In the driving method for developing such bi-stable state as shown in Figs. 8 and 10, a hysteresis curve showing a favorable double state stability can be formed, for example, by varying the applied voltage between -30 V and +30 V. In the liquid crystal element showing a triple state stability, an electric field can be applied in the manner similar to that of the above-mentioned case.

Further, to this liquid crystal element may be applied an electric field having the above-mentioned wave form by varying it between 0 and a positive voltage. Namely, a light modulation method utilizing, for example, light transmission properties shown by a hysteresis curve formed at a plus voltage side by application of an electric field varied in voltage by varying a voltage in the range between 0 and +30 V can be employed. Similarly, a light modulation method utilizing light transmission properties shown by a hysteresis curve formed at a minus voltage side by application of an electric field varied in voltage by

varying a voltage in the range between 0 to -30 V can be employed.

The liquid crystal element of the invention is superior to the prior art liquid crystal elements in that the present element can be driven by utilizing two kinds of driving methods as mentioned previously, and it can retain its memory effect by suitably selecting a desired driving method out of the two methods according to the conditions under which it is driven.

The liquid crystal element as mentioned above may be used in applications to which ordinary liquid crystal elements are applied, but said liquid crystal element is particularly useful as a display element.

The display element includes, for example, a liquid crystal large frame display, multi-information display for use in car, navigation display for use in car and display for laptop personal computer. These display elements can be driven, according to the purposes for which they are used, by the above-mentioned driving methods as a bi-stable state liquid crystal element or tri-stable state type liquid crystal element.

The following methods may be given as examples of the display or driving method in which the present liquid crystal elements are used.

The first display or driving method is to effect the display by placing the liquid crystal element of the invention between two polarizing plates and applying an external electric field to said element to change the orientation vector of the ferroelectric or anti-ferroelectric liquid crystal composition present in the element, thereby effecting said display by utilizing a birefringence of the two polarizing plates and of the ferroelectric or anti-ferroelectric liquid crystal composition.

The second display or driving method using the liquid crystal element of the invention comprises using as a liquid crystal material a liquid crystal composition containing a dichroic dye and utilizing the dichroism of the dye. This second method is to effect the display by changing light absorption wavelength by means of the dye while changing the orientation direction of the molecules in the ferroelectric or anti-ferroelectric liquid crystal compound. In this case, the dyes used are usually dichroic dyes, and examples of the dichroic dyes include azo dyes, naphthoquinone dyes, cyanine dyes and anthraquinone dyes.

The liquid crystal elements of the invention may be applicable to commonly used display methods in addition to the above-mentioned methods.

The display devices prepared by using the liquid crystal elements of the invention may be driven by various driving methods, for example, electric address display such as static drive, simple matrix drive and composite matrix drive, and photo-address display, heat address display and electron beam address display.

## EFFECT OF THE INVENTION

The carboxylate compounds of the present invention are novel compounds.

These novel carboxylate compounds are optically active, in which 1,2,3,4-tetrahydronaphthylene ring and benzene ring are linked through ester linkage, and two benzene rings, if any, are also linked together through ester linkage. Hence, these compounds assume the smectic phase over a wide temperature range and can be preferably used as the ferroelectric liquid crystal compounds.

By virtue of mixing the liquid crystal compounds of the invention with the same kinds of liquid crystal compounds and/or different kinds of liquid crystal compounds, the temperature range within which the liquid crystal element can be used may be broadened without marring the ferroelectric or anti-ferroelectric properties of the liquid crystal compounds of the invention.

Accordingly, liquid crystal elements having a high speed response over a wide temperature range can be obtained by using the above-mentioned liquid crystal compounds or liquid crystal compositions.

Further, scanning time is markedly shortened in the liquid crystal display devices prepared by using such elements as mentioned above.

When such display devices are used, power consumption can be reduced, a stable contrast can be obtained and also a low voltage driving can be performed.

By way of the liquid crystal elements of the invention and the light modulation method using said liquid crystal elements, it becomes possible to attain steady state in two forms, i.e. bi-stable state and tri-stable state.

When the liquid crystal elements of the invention are used, a dark state having a sufficient darkness can be attained, and hence a very high contrast between the bright and dark states can be obtained and, at the same time, a favorable memory effect can be secured.

The present invention is illustrated below with reference to examples, but it should be construed that the invention is in no way limited to those examples.

Example 1

Synthesis of R-1'''-trifluoromethylheptyl ester of 4-[4'-(1'',2'',3'',4''-tetrahydro-6''-n-decyloxy-2''-naphthoyloxy) benzoyloxy]-benzoic acid

First step

To a mixture of 86 g (11.8 mmol) of 6-n-decyloxynaphthalene-2-carboxylic acid and 130 ml of 1,2-diethoxyethane was added in a nitrogen atmosphere at 120 °C with stirring 3.0 g (130 mg atom) of metallic sodium, and the mixture was then heated to a refluxing temperature.

To this mixture was added dropwise 10 g (114 mmol) of iso-amyl alcohol, and the mixture was allowed to undergo reaction for 1 hour under reflux. After cooling the reaction mixture to room temperature, the metallic sodium remaining in the mixture is decomposed by the addition of ethanol, and the reaction mixture was then acidified with 20% hydrochloric acid.

After addition of 100 ml of water to this reaction mixture, an organic layer was separated therefrom, and this organic layer was washed with water.

The organic layer was concentrated under reduced pressure to obtain 4.25 g of a solid. The solid was recrystallized from toluene to obtain 2.95 g (8.89 mmol) of 1,2,3,4-tetrahydro-6-n-decyloxynaphthalene-2-carboxylic acid.

Second step

To a mixture of 1.66 g (5 mmol) of 1,2,3,4-tetrahydro-6-n-decyloxynaphthalene-2-carboxylic acid obtained in the first step, 1.14 g (5 mmol) of benzyl 4-hydroxybenzoate, 0.12 g (1 mmol) of 4-N,N-dimethylaminopyridine and 20 ml of methylene chloride was added dropwise with stirring at room temperature over a period of one hour.

The reaction was carried out at room temperature for additional 10 hours.

The reaction mixture was filtered and the filtrate was concentrated. Using column chromatography, 2.32 g (4.28 mmol) of benzyl 4-(1',2',3',4'-tetrahydro-6'-n-decyloxy-2'-naphthoyloxy)benzoate as a white solid was separated from the concentrate.

Third step

Hydrogen gas was passed through a mixture of 2.17 g (4 mmol) of benzyl 4-(1',2',3',4'-tetrahydro-6'-n-decyloxy-2'-naphthoyloxy)benzoate obtained in the second step, 1 g of 5% palladium supported on carbon and 30 ml of tetrahydrofuran with stirring at room temperature and ordinary pressure for 8 hours. The reaction mixture was filtered by using Celite which is a filtration assistant and the filtrate obtained was concentrated to obtain 1.59 g (3.52 mmol) of 4-(1',2',3',4'-tetrahydro-6'-n-decyloxy-2'-naphthoyloxy)benzoic acid as a white solid.

Fourth step

To a mixture of 0.45 g (1 mmol) of 4-(1',2',3',4'-tetrahydro-6'-n-decyloxy-2'-naphthoyloxy)benzoic acid obtained in the third step, 0.30 g (1 mmol) of R-1'-trifluoromethylheptyl-4-hydroxybenzoate, 0.012 g (0.1 mmol) of 4-N,N-dimethylaminopyridine and 10 ml of methylene chloride was added dropwise 2 ml of methylene chloride solution containing 0.21 g (0.1 mmol) of N,N'-dicyclohexylcarbodiimide with stirring at room temperature over a period of one hour. The mixture was allowed to undergo reaction at room temperature for 8 hours. The reaction mixture was filtered, and the filtrate obtained was concentrated. The concentrate was separated by using column chromatography to obtain 0.52 g of a colorless semi-solid.

FD-mass spectrum of this semi-solid was measured to obtain an M/e value of 738.

Fig. 1 shows a chart of $^1$H-NMR spectrum of this compound.

From these results of the analyses, the compound was identified to be R-1'''-trifluoromethylheptyl 4-[4'-(1'',2'',3'',4''-tetrahydro-6''-n-decyloxy-2''-naphthoyloxy)benzoyloxy]benzoate which was the desired compound [Exemplified compound (5)].

Example 2

Synthesis of R-1''-trifluoromethylheptyl 4-(1',2',3',4'-tetrahydro-6'-n-decyloxy-2'-naphthoyloxy)benzoate

Fifth step

To a mixture of 0.33 g (1 mmol) of 1,2,3,4-tetrahydro-6-n-decyloxy-naphthalene-2-carboxylic acid obtained in the first step, 0.30 g (1 mmol) of R-1'-trifluoromethylheptyl 4-hydroxybenzoate, 0.012 g (0.1 mmol) of 4-N,N-dimethylaminopyridine and 10 ml of methylene chloride was added dropwise 2 ml of methylene chloride solution containing 0.21 g (1 mmol) of N,N-dicyclohexylcarbodiimide with stirring at room temperature over a period of one hour.

The reaction was carried out at room temperature for 8 hours.

The reaction mixture was filtered and the filtrate obtained was concentrated.

The concentrate was separated by using column chromatography to obtain 0.58 g of a colorless viscous liquid.

FD-mass spectrum of this compound was measured to obtain a M/e value of 618.

Fig. 2 shows a chart of $^1$H-NMR spectrum of this compound.

From these results of the analyses, this compound was identified to be R-1''-trifluoromethylheptyl 4-(1',2',3',4'-tetrahydro-6'-n-decyloxy-2'-naphthoyloxybenzoate which was the desired compound [Exemplified compound (13)].

Example 3

Synthesis of R-1'''-trifluoromethylheptyl 4-[4'-(1'',2'',3'',4''-tetrahydro-6''-n-heptyloxy-2''-naphthoyloxy)-benzoyloxy]benzoate

Example 1 was repeated except that in place of the 6-n-decyloxy-naphthalene-2-carboxylic acid used in the first step, 6-n-heptyloxy-naphthalene-2-carboxylic acid was used to obtain 0.34 g of a colorless semi-solid.

FD-mass spectrum of this semi-solid was measured to obtain an M/e value of 696.

Fig. 3 shows a chart of $^1$H-NMR of this compound.

From these result of the analyses, this compound was identified to be the desired R-1'''-trifluoromethylheptyl 4[4'-(1'',2'',3'',4''-tetrahydro-6''-n-heptyloxy-2''-naphthoyloxy)benzoyloxy]benzoate [Exemplified compound (8)].

Example 4

Phase transition temperatures of Exemplified compound (1), (5), (8) and (13) obtained in Examples 1 and 2, respectively were measured.

Results obtained are shown in Table 3.

Table 3

| Compound | Phase transition temperature | | |
|---|---|---|---|
| | Cry-SmC* or SmA | SmC*-SmA | SmA-Iso |
| (1) | 30 °C | | 72 °C |
| (5) | 44 °C | 78 °C | 94 °C |
| (8) | <-30°C | 47 °C | 105 °C |
| (13) | -14 °C | | |

In Table 3, Cry represents a crystal phase, SmC* represents a chiral smectic C phase, SmA represents a smectic A phase, and Iso represents an isotropic liquid.

As is clear from Table 3, the compound (5) and compound (13) assumed a liquid crystal phase over a wide temperature range or below room temperature.

Subsequently, the above-mentioned carboxylate compound (5) and a compound represented by the following formula [B] were mixed together in a ratio of 50:50 by weight to prepare a liquid crystal composition according to the present invention.

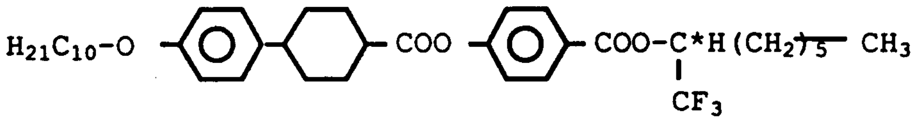

...[B]

A phase transition temperature of this composition was measured. Results obtained are shown in Table 4. The phase transition temperature of the above-mentioned compound of the formula [B] is also shown in Table 4.

Table 4

| Compound or composition | Phase transition temperature | | |
|---|---|---|---|
| | CRY-SmC* | SmC-SmA* | SmA-Iso or SmC* |
| [5] | 44 °C | 78 °C | 94 °C |
| [5] 50wt% + (B) 50wt% | <-30 °C | 50 °C | 72 °C |
| (B) | 26 °C | | 53 °C |

⟨Notes⟩ In the above table, the compound [5] has the following formula

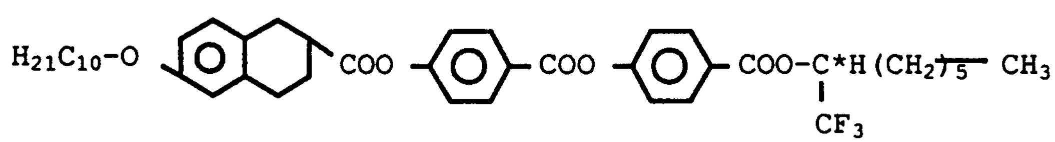

and the compound (B) has the following formula.

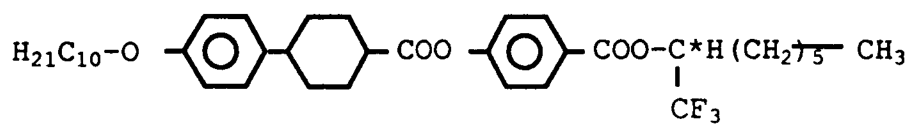

Example 5

A liquid crystal element was prepared by filling the liquid crystal composition into a cell shown in Fig. 6-(A).

The operable temperature of the liquid crystal element was from 72 °C to -30 °C and the contrast of the element was stable in the temperature range.

Example 6

The carboxylate compound [5] of the formula [5] shown in Table 4 was molted and injected into a gap of a cell, said gap being kept under reduced pressure and said cell being composed of two substrates with ITO transparent electrodes, each substrate being provided with an orientation controlling film (thickness : 150 Å ) composed of a polyimide (LX1400, a product of Hitachi Kasei Kogyo K.K.) on the inner surface thereof as shown in Fig. 6(A). The polyimide film was rubbed so that orientation directions were nearly parallel to each other and in the same direction. The cell thus filled with the liquid crystal material wad heated to 120 °C, kept at 120 °C for 5 minutes and cooled at a rate of 1 °C/min to 60 °C to prepare a liquid crystal element.

The contrast of the liquid crystal element was measured. The contrast was 20.

Cell condition :

(a) External size : 2.5 cm long X 2.2 cm width X 1.5 mm thick
(b) Substrate : 0.7 mm thick, material : glass
(c) Distance between substrates : 2 μm
(d) Sidewall size : 1.8 mm long X 0.1 cm width X 2 μm thick

The above cell used for evaluation of liquid crystal was prepared in the following manner.

Polyimide coating was conducted on a glass substrate with ITO transparent electrode film. That is, the polyimide (LX1400, a product of Hitachi Kasei Kogyo K.K.) was applied on the ITO transparent electrode by a spin coating method. The polyimide was diluted with N-methylpyrrolidone to a 1.2 % solution which was then spin-coated at 2000 rpm. The polyimide solution thus coated was cured by heating at 325 °C for 30 minutes, whereupon a polyimide film of 150 to 200 Å in thickness was formed. The polyimide film was then rubbed with a nylon cloth in one direction, thereby imparting an ability of orientating the liquid crystal thereto.

Two sheets of the polyimide film-coated glass substrate thus prepared were put upon each other to prepare a cell for evaluation. An epoxy adhesive was applied to each of the polyimide film-coated glass substrates by means of silk screen printing so that two substrates were bonded to each other and a gap of the cell was controlled. The epoxy adhesive was prepared by mixing an adhesive base (LCB-310B, a product of EHC) with a curing agent (LCB-304B, a product of EHC) and beads (GP-20, a product of EHC) for controlling cell gas in the proportion of 130:30:3. One of the glass substrates mentioned above was coated with the epoxy adhesive and laminated to other glass substrate in such a manner that the polyimide films faced each other. The epoxy adhesive thus coated was cured under such curing conditions that heating was conducted at 50 °C for 15 minutes, at 60 °C for 15 minutes, at 70 °C for 15 minutes, at 80 °C for 15 minutes, at 125 °C for 30 minutes and at 170 °C for 60 minutes.

The liquid crystal material was evaluated by using the thus prepared cell for evaluation having a gap of about 2 μm.

In the present invention, contrast was determined by placing the liquid crystal material between polarizers meeting at right angles, measuring an intencity of transmitted light in the light state and in the dark state by rotating the liquid crystal element, and calculating therefrom the ratio of I (light state)/I (dark state).

Example 7

A liquid crystal element was prepared by repeating Example 6 except that the orientation directions of the orientation controlling films composed of polyimide were nearly parallel but in the opposite direction from each other.

The contrast of the thus obtaine liquid crystal element as measured was 18.

Example 8

A liquid crystal element was prepared by repeating Example 6 except that one of the substrates was prepared by forming a rhombic deposited film composed of silicon oxide on the glass substrate provided with ITO transparent electrode film.

The rhombic deposited film was formed by heating $SiO_2$ to 400 °C and depositing it on the substrate from the vertical direction, the substrate being inclined at 30° from a horizontal plane.

In the liquid crystal element thus prepared, the orientation direction of the rhombic deposited film and that of the orientation controlling film were nearly parallel but in the opposite direction from each other.

The contrast of the thus obtained liquid crystal element as measured was 17.

Example 9

A liquid crystal element was prepared by repeating Example 6 except that the cooling rate was changed to 0.1 °C/min.

The contrast of the thus obtained liquid crystal element was 29.

Example 10

A liquid crystal element was prepared by repeating Example 6 except that the liquid crystal composition obtained in Example 4 was used in place of the carboxylate compound and the cooling rate was changed to 0.1 °C/min.

The contrast of the thus obtained liquid crystal element was 21.

Example 11

A liquid crystal element was prepared by repeating Example 6 except that the cooling rate was changed to 10 °C/min.

The contrast of the thus obtained liquid crystal element was 9. Because of rapid cooling rate employed, it was observed that the contrast was apt to be somewhat low.

Example 12

The carboxylate compound [5] of the formula [5] shown in Table 4 was molten and injected into a gap of a cell, said gap being kept under reduced pressure and said cell being composed of two substrates with ITO transparent electrodes, each substrate being provided with an orientation controlling film (thickness: 150 Å) composed of a polyimide (LX1400, a product of Hitachi Kasei Kogyo K.K.) on the inner surface thereof as shown in Fig. 6(B). The polyimide film was rubbed so that orientation directions were nearly parallel to each other and in the same direction. The cell thus filled with the liquid crystal material was heated to 120 °C, kept at 120 °C for 5 minutes and cooled to 60 °C at a rate of 1 °C/min to prepare a liquid crystal element.

Cell condition:

(a) External size: 2.5 cm long x 2.2 cm width x 1.5 mm thick
(b) Substrate: 0.7 mm thick, material: glass
(c) Distance between substrates: 2 µm
(d) Sidewall size: 1.8 mm long x 2.2 mm width x 1.5 µm thick

The above-mentioned cell was prepared in the same manner as in Example 6.

A liquid crystal element was prepared by placeing the above-mentioned liquid crystal cell filled with the liquid crystal material between two polarizing plates whose planes of polarization meet at right angles so that the darkest state is attained in the element.

The intencity of transmitted light was measured by applying a triangular wave of $30V_{p-p}$, whereupon the oscillowave shown in Fig. 7 was obtained by application of a frequency of 10 Hz, and the oscillowave shown in Fig. 8 was obtained by application of a frequency of 100 Hz.

As is clear from Fig. 7, this liquid crystal element attained the contrast of 34 between the time when 0 V was applied and the time when +30 V (or -30 V) was applied by application of a triangular wave of 10 Hz.

As is clear from Fig. 8, this liquid crystal element attained the contrast of 15 between the time when -12 V was applied and the time when +12 V was applied by application a triangular wave of 100 Hz.

In the liquid crystal element of the invention in which the liquid crystal cell is placed between two polarizing plates whose planes of polarization meet at right angles so that the darkest state is attained in the element, the dark state can be attained by applying a voltage of 0 V using a low frequency, in particular.

The contrast mentioned above was determined by measuring the intencity of the transmitted light in the light state and in the dark state while changing a voltage applied to the liquid crystal element, and calculating therefrom the ratio of I (light state)/I (dark state).

Subsequently, a liquid crystal element was prepared by placing the above-mentioned liquid crystal cell filled with the liquid crystal material between two polarizing plates whose planes of polarization meet at right

angles so that the lightest state is attained in the element.

The intencity of transmitted light was measured by applying a triangular wave of 30 $V_{p-p}$, whereupon the oscillowave shown in Fig. 9 was obtained by application of a frequency of 10 Hz, and the oscillowave shown in Fig. 10 was obtained by application of a frequency of 100 Hz.

From the above results, it was found that the liquid crystal element in which the liquid crystal cell is placed between two polarizing plates whose planes of polarization meet at right angles so that the lightest state is attained in the element could secure a favorable memory effect using a high frequency, in particular.

## Claims

**1.** A compound of the following formula (A):

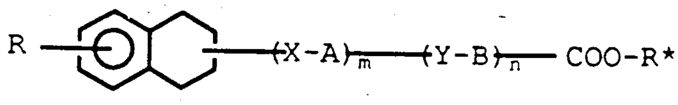 ... [A]

wherein R is a group selected from $C_3$-$C_{20}$ alkyl, $C_3$-$C_{20}$ alkoxy and halogenated $C_3$-$C_{20}$ alkyl, X and Y are each, independently, a group selected from:
-COO-,-OCO-, $-CH_2CH_2-$. $CH_2O$-, $OCH_2$-, -S-S-,

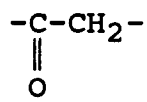

and

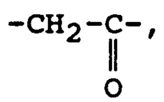

or a single bond, A and B are each, independently, a group selected from

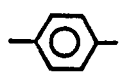, 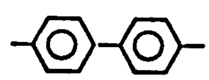,

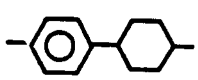, 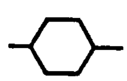, 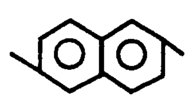, 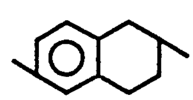, 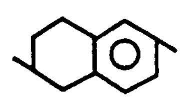,

and

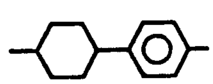

and R* is an optically active group of 4 to 20 carbon atoms, the group containing at least one asymmetric carbon atom and being optionally substituted by one or more halogen atoms, and m and n are each an integer of 0 to 2; with the proviso that both m and n are not 0 at the same time.

**2.** A compound according to claim 1 wherein X and Y are each selected from -COO-, -OCO-, $-CH_2CH_2-$, $-CH_2O-$ and $-OCH_2-$.

3. A compound according to claim 1 or 2 wherein the optically active group R* is selected from

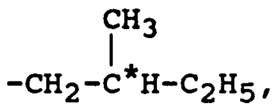 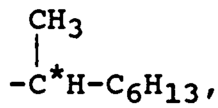 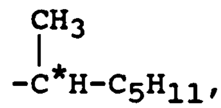 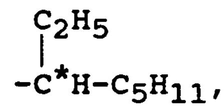

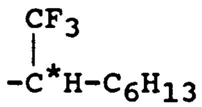 and 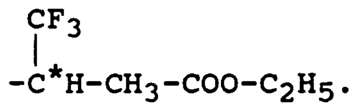

4. A compound according to any one of claims 1 to 3 wherein R is alkoxy, X is -COO-, A is

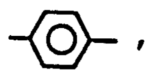

R* is

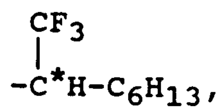

m is an integer of 1 or 2, and n is 0.

5. A liquid crystal composition which comprises a compound of formula (A) as claimed in any one of claims 1 to 4.

6. A liquid crystal composition according to claim 5 wherein the compound of formula (A) is contained in an amount of 1 to 99 parts by weight based on 100 parts by weight of the composition.

7. A liquid crystal element which comprises a cell composed of two substrates facing each other and separated by a gap, the gap being filled with a liquid crystal material comprising a liquid crystal composition as claimed in claim 5 or 6.

8. A liquid crystal element according to claim 7 wherein an orientation controlling film is provided on the inner surface of at least one substrate.

9. A liquid crystal element according to claim 8 wherein the orientation controlling film is an orientation controlling film that has been subjected to orientation treatment.

10. A liquid crystal element according to any one of claims 7 to 9 wherein each substrate is provided on its inner surface with a transparent electrode and wherein a polarizing plate is provided on the outside of each substrate such that a plane of polarization formed by the polarizing plates has an angle of 70°-110°, and the cell filled with the liquid crystal material is arranged between the polarizing plates at an angle of from +10° to -10° relative to the position of the cell at which the transmitted light becomes the darkest or the brightest.

11. A process for the preparation of a liquid crystal element as claimed in any one of claims 7 to 10, which process comprises forming the cell by providing an orientation controlling film on the inner surface of at least one substrate, filling the gap with the liquid crystal material, heating the liquid crystal material contained in the cell to a temperature not lower than the temperature at which the material exhibits the properties of an isotropic liquid, and cooling the material to a temperature not higher than the temperature at which the material exhibits the properties of a liquid crystal.

12. A process according to claim 11 wherein the liquid crystal material is cooled at a cooling rate of not higher than 2 ° C/min from a temperature higher than the temperature at which the crystal material exhibits the properties of an isotropic liquid to a temperature lower than the temperature at which the material exhibits the properties of a liquid crystal.

13. A method of liquid modulation which comprises generating an electric field on, or applying a voltage to, a liquid crystal element as claimed in claim 10.

14. A method according to claim 13 wherein the amount of light transmitted by the liquid crystal element is changed by varying the voltage applied to the element between a positive voltage and a negative voltage.

15. A method according to claim 13 or 14 wherein the wave form of the electric field generated by varying the voltage applied to the liquid crystal element between a positive voltage and a negative voltage is a rectangular wave, triangular wave, sine wave or a combination thereof.

16. A method according to claim 15 wherein, when the wave form is a rectangular wave, the minimum width of the rectangular wave is not more than 10 m sec.

17. A method according to any one of claims 13 to 16 wherein the amount of light transmitted by the liquid crystal element is changed by varying the voltage applied to the element from 0 to a positive voltage, or from 0 to a negative voltage.

18. A display unit, a liquid crystal display device, an eletrooptical display device or a light modulation element, comprising a liquid crystal element as claimed in any of claims 7 to 10.

19. A display method which includes the method of light modulation as claimed in any of claims 13 to 17.

**Patentansprüche**

1. Verbindung der folgenden Formel (A):

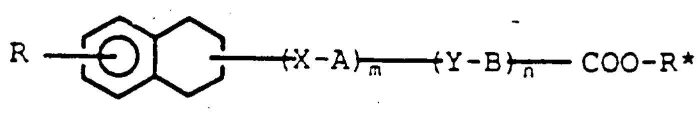

... [A]

worin R eine aus $C_3$-$C_{20}$-Alkyl-, $C_3$-$C_{20}$-Alkoxy und halogeniertem $C_3$-$C_{20}$-Alkyl ausgewählte Gruppe ist, X und Y unabhänig voneinander jeweils eine aus -COO-, -OCO-, $-CH_2CH_2$, $CH_2O$-, $OCH_2$-, -S-S-,

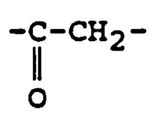

und

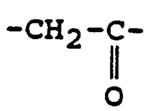

gewählte Gruppe oder eine Einfachbindung bedeuten, A und B unabhängig voneinander jeweils eine aus

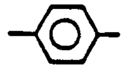, 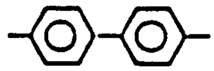,

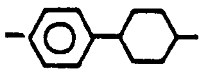, 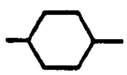, 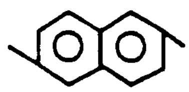, 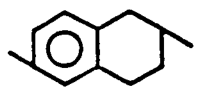, 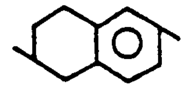,

und

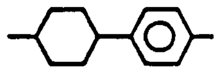

gewählte Gruppe bedeuten und R* eine optisch aktive Gruppe mit 4 bis 20 Kohlenstoffatomen ist, wobei die Gruppe mindestens ein asymmetrisches Kohlenstoffatom enthält und gegebenenfalls durch ein oder mehrere Halogenatome substituiert ist, umd m und n jeweils eine ganze Zahl von 0 bis 2 sind; mit der Maßgabe, daß m und n nicht gleichzeitig 0 sind.

**2.** Verbindung gemäß Anspruch 1, worin X und Y jeweils aus -COO-, -OCO-, $-CH_2CH_2$, $CH_2O$-, $OCH_2$-gewählt werden.

**3.** Verbindung gemäß Anspruch 1 oder 2, worin die optisch aktive Gruppe R* aus folgendem ausgewählt wird:

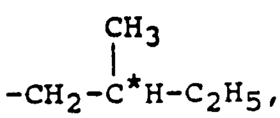, 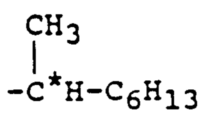, 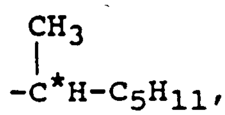, 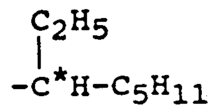,
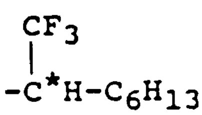 and 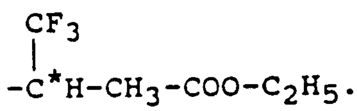.

**4.** Verbindung gemäß mindestens einem der Ansprüche 1 bis 3, worin R Alkoxy ist, X -COO- ist, A für

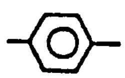

steht, R*

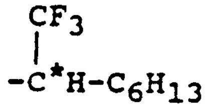

bedeutet, m eine ganze Zahl von 1 oder 2 ist und n für 0 steht.

**5.** Flüssigkristallzusammensetzung, welche eine Verbindung der Formel (A) gemäß einem der Ansprüche 1 bis 4 umfaßt.

**6.** Flüssigkristallzusammensetzung gemäß Anspruch 5, worin die Verbindung der Formel (A) in einer Menge von 1 bis 99 Gewichtsteilen, bezogen auf 100 Gewichtsteile der Zusammensetzung, enthalten ist.

**7.** Flüssigkristallelement, welches eine Zelle umfaßt, die aus zwei sich gegenüberstehenden, und durch eine Lücke getrennten Substraten besteht, wobei die Lücke mit einem Flüssigkristallmaterial gefüllt ist, das eine Flüssigkristallzusammensetzung nach Anspruch 5 oder 6 umfaßt.

**8.** Flüssigkristallelement gemäß Anspruch 7, worin ein Orientierungsregulationsfilm an der Innenoberfläche von mindestens einem Substrat vorgesehen ist.

**9.** Flüssigkristallelement gemäß Anspruch 8, wobei der Orientierungsregulationsfilm ein Orientierungsregulationsfilm ist, der einer Orientierungsbehandlung unterzogen wurde.

**10.** Flüssigkristallelement gemäß mindestens einem der Ansprüche 7 bis 9, wobei jedes Substrat an seiner Innenoberfläche mit einer transparenten Elektrode versehen ist, und wobei eine Polarisationsplatte an der Außenseite jedes Substrates vorgesehen ist, so daß eine durch die Polarisationsplatten gebildete Polarisationsebene einen Winkel von 70° - 110°C besitzt und die mit die Flüssigkristallmaterial gefüllte Zelle zwischen den Polarisationsplatten in einem Winkel +10° bis -10°, bezogen auf die Position der Zelle, bei der das durchgelassene Licht am dunkelsten oder am hellsten wird, angeordnet ist.

**11.** Verfahren zur Herstellung eines Flüssigkristallelementes nach mindestens einem der Ansprüche 7 bis 10, welches Verfahren das Ausbilden der Zelle unter Vorsehen eines Orientierungsregulationsfilmes auf der Innenoberfläche von mindestens einem Substrat, das Füllen der Lücke mit dem Flüssigkristallmaterial, der Erwärmen des in der Zelle enthaltenen Flüssigkristallmaterials auf eine Temperatur, die nicht geringer als die Temperatur ist, bei der das Material die Eigenschaften einer isotropen Flüssigkeit zeigt, und das Abkühlen des Materials auf eine Temperatur, die nicht höher ist als die Temperatur, bei der das Material die Eigenschaften eines Flüssigkristalls zeigt, umfaßt.

**12.** Verfahren gemäß Anspruch 11, worin das Flüssigkristallmaterial mit einer Abkühlungsgeschwindigkeit von nicht mehr als 2°C/min von einer Temperatur, die höher als die Temperatur ist, bei der das Kristallmaterial die Eigenschaften einer isotropen Flüssigkeit zeigt, auf eine Temperatur, die niedriger als die Temperatur ist, bei der das Material die Eigenschaften eines Flüssigkristalls zeigt, abgekühlt wird.

**13.** Verfahren zur Flüssigkeitsmodulation, welches die Erzeugung eines elektrischen Feldes auf oder die Anwendung einer Spannung an ein Flüssigkristallelement gemäß Anspruch 10 umfaßt.

**14.** Verfahren nach Anspruch 13, wobei die Menge des durch das Flüssigkristallelement hindurchgelassenen Lichtes verändert wird, indem die an das Element angelegte Spannung zwischen einer positiven Spannung und einer negativen Spannung variiert wird.

**15.** Verfahren gemäß Anspruch 13 oder 14. wobei die Wellenform des elektrischen Feldes, welches durch Verändern der an das Flüssigkristallelement angelegten Spannung zwischen einer positiven Spannung und einer negativen Spannung erzeugt wird, eine Rechteckwelle, Dreieckwelle, Sinuswelle oder eine Kombination davon ist.

**16.** Verfahren gemäß Anspruch 15, wobei die minimalste Breite der Rechteckwelle nicht mehr als 10 m sec beträgt, wenn die Wellenform eine Rechteckwelle ist.

**17.** Verfahren gemäß mindestens einem der Ansprüche 13 bis 16, wobei die Menge des durch das Flüssigkristallelement hindurchgelassenen Lichtes verändert wird, indem die an das Element angelegte Spannung zwischen 0 und einer positiven Spannung oder zwischen 0 und einer negativen Spannung variiert wird.

**18.** Anzeigeeinheit, Flüssigkristallanzeigevorrichtung, elektrooptische Anzeigevorrichtung oder ein Lichtmodulationselement, umfassend ein Flüssigkristallelement gemäß mindestens einem der Ansprüche 7 bis 10.

**19.** Anzeigeverfahren, welches das Verfahren der Lichtmodulation gemäß mindestens einem der Ansprüche 13 bis 17 umfaßt.

## Revendications

1. Composé de formule suivante (A):

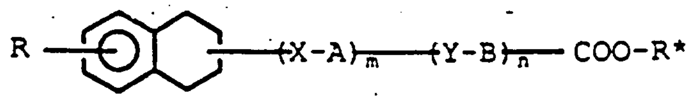 ... [A]

dans laquelle R représente un groupe choisi parmi les groupes alkyle en $C_{3-20}$, alcoxy en $C_{3-20}$ et alkyle en $C_{3-20}$ halogéné, X et Y représentent chacun, indépendamment, une simple liaison ou un groupe choisi parmi -COO-, -OCO-, $-CH_2CH_2-$, $-CH_2O-$, $-OCH_2-$, -S-S-, $-CO-CH_2-$ et $-CH_2-CO-$, A et B représentent chacun, indépendamment, un groupe choisi parmi

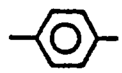, 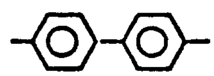, 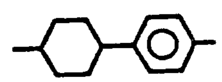,

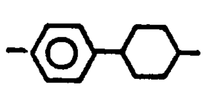, 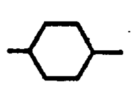, 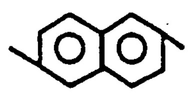, 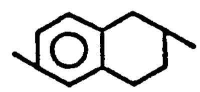, 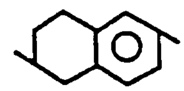,

et R* représente un groupe optiquement actif comportant de 4 à 20 atomes de carbone, ce groupe contenant au moins un atome de carbone asymétrique et portant éventuellement un ou plusieurs atomes d'halogène en tant que substituants, et m et n représentent chacun un nombre entier valant de 0 à 2, pourvu que m et n ne vaillent pas tous les deux zéro en même temps.

2. Composé conforme à la revendication 1, dans lequel X et Y sont chacun choisis parmi -COO-, -OCO-, $-CH_2CH_2-$, $-CH_2O-$ et $-OCH_2-$.

3. Composé conforme à la revendication 1 ou 2, dans lequel le groupe optiquement actif R* est choisi parmi

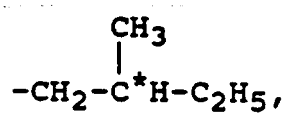 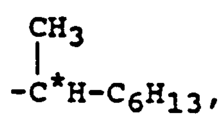 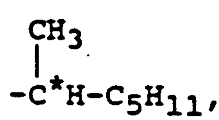 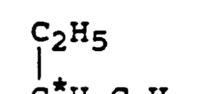
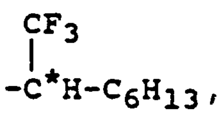 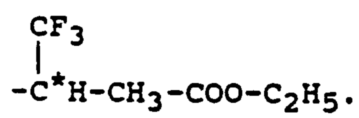

4. Composé conforme à l'une des revendications 1 à 3, dans lequel R représente un groupe alcoxy, X représente -COO-, A représente un groupe para-phénylène, R* représente $-C^{*}H(CF_3)(C_6H_{13})$, m représente un nombre entier valant 1 ou 2, et n vaut 0.

5. Composition de cristal liquide, qui comprend un composé de formule (A) conforme à l'une des revendications 1 à 4.

6. Composition de cristal liquide, conforme à la revendication 5, qui contient de 1 à 99 parties en poids de composé de formule (A) pour 100 parties en poids de composition.

7. Elément à cristal liquide, qui comprend une cellule constituée de deux substrats disposés l'un en face de l'autre et séparés par un espace, lequel espace est rempli d'une matière à cristal liquide, comprenant une composition de cristal liquide conforme à la revendication 5 ou 6.

8. Elément à cristal liquide, conforme à la revendication 7, dans lequel un film de commande d'orientation est placé sur la face interne d'au moins l'un des substrats.

9. Elément à cristal liquide, conforme à la revendication 8, dans lequel le film de commande d'orientation est un film de commande d'orientation qui a subi un traitement d'orientation.

10. Elément à cristal liquide, conforme à l'une des revendications 7 à 9, dans lequel chaque substrat est muni d'une électrode transparente placée sur sa face interne, et dans lequel un polariseur est disposé sur la face externe de chacun des substrats, de telle sorte que les plans de polarisation associés aux polariseurs forment un angle de 70° à 110°, et la cellule remplie de matière à cristal liquide est disposée, entre les polariseurs, dans une position faisant un angle de +10° à -10° par rapport à la position de la cellule pour laquelle la lumière transmise est le moins intense ou le plus intense.

11. Procédé de fabrication d'un élément à cristal liquide conforme à l'une des revendications 7 à 10, lequel procédé comprend le fait de former une cellule tout en plaçant un film de commande d'orientation sur la face interne d'au moins un substrat, le fait de remplir l'espace de la cellule avec la matière à cristal liquide, le fait de chauffer la matière à cristal liquide contenue dans la cellule à une température au moins égale à celle à laquelle cette matière présente les propriétés d'un liquide isotrope, et le fait de refroidir cette matière à une température au plus égale à celle à laquelle cette matière présente les propriétés d'un cristal liquide.

12. Procédé conforme à la revendication 11, dans lequel on refroidit la matière à cristal liquide, à une vitesse de refroidissement d'au plus 2°C/min, depuis une température supérieure à celle à laquelle cette matière présente les propriétés d'un liquide isotrope jusqu'à une température inférieure à celle à laquelle cette matière présente les propriétés d'un cristal liquide.

13. Procédé de modulation de lumière, qui comprend le fait d'établir un champ électrique dans un élément à cristal liquide conforme à la revendication 10, ou d'appliquer une tension électrique à un tel élément.

14. Procédé conforme à la revendication 13, dans lequel on fait varier la quantité de lumière transmise par l'élément à cristal liquide en faisant varier la tension appliquée à cet élément entre une valeur positive et une valeur négative.

15. Procédé conforme à la revendication 13 ou 14, dans lequel l'onde de champ électrique créée par la variation de la tension appliquée à l'élément à cristal liquide entre une valeur positive et une valeur négative est une onde rectangulaire, une onde triangulaire, une onde sinusoïdale ou une combinaison de telles ondes.

16. Procédé conforme à la revendication 15, dans lequel l'onde est une onde rectangulaire dont la largeur minimale ne vaut pas plus de 10 millisecondes.

17. Procédé conforme à l'une des revendications 13 à 16, dans lequel on fait varier la quantité de lumière transmise par l'élément à cristal liquide en faisant varier la tension appliquée à cet élément de zéro à une valeur positive, ou de zéro à une valeur négative.

18. Unité d'affichage, dispositif d'affichage à cristaux liquides, dispositif d'affichage électro-optique ou élément à modulation de lumière, comprenant un élément à cristal liquide conforme à l'une des revendications 7 à 10.

19. Procédé d'affichage qui fait appel à un procédé de modulation de lumière conforme à l'une des revendications 13 à 17.

FIG. 1

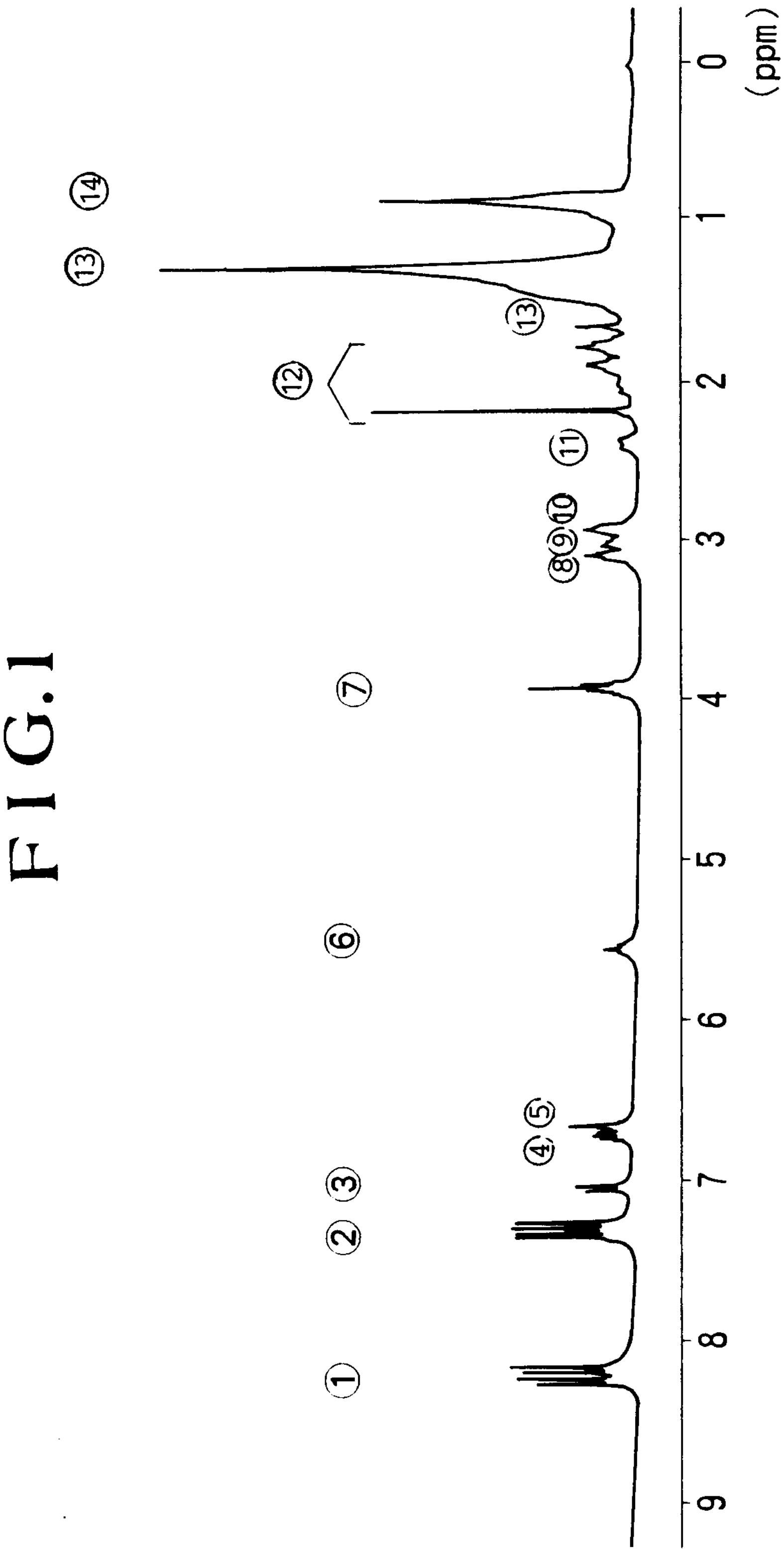

FIG.2

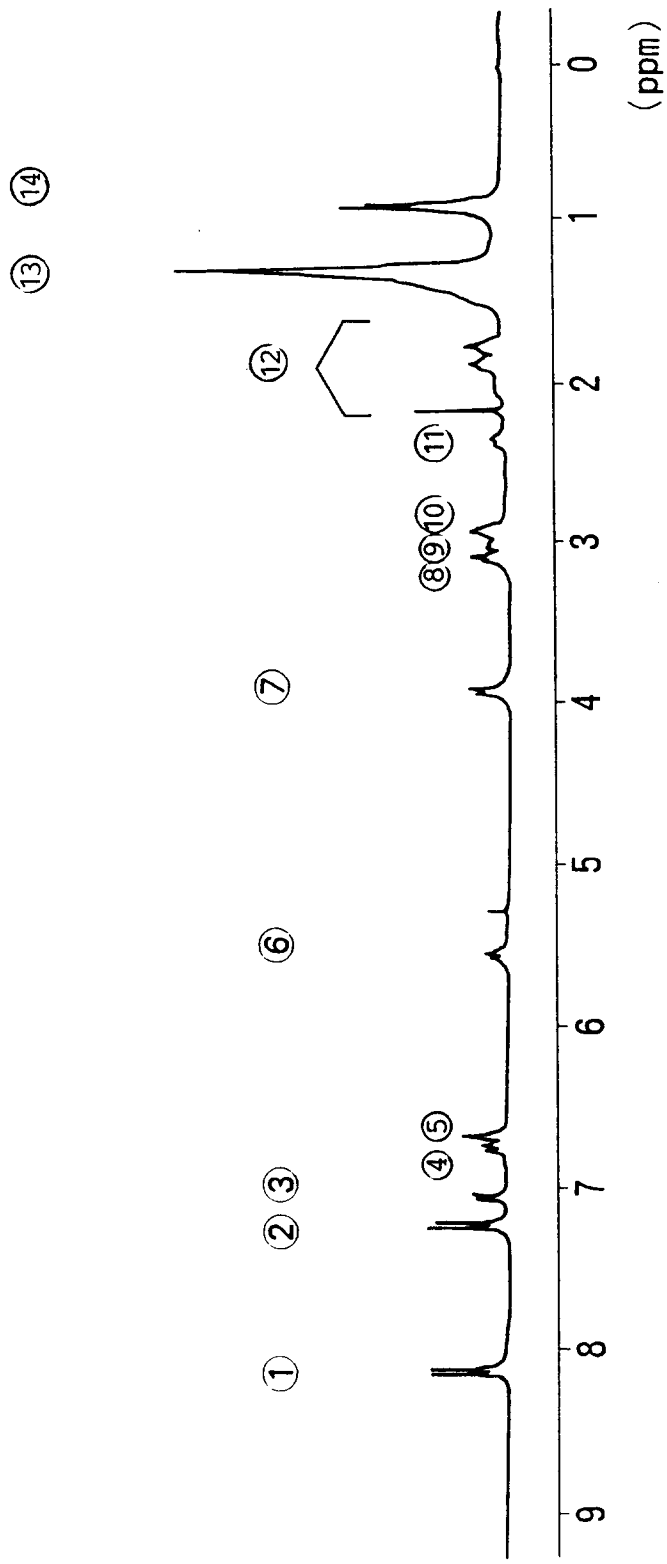

FIG.3

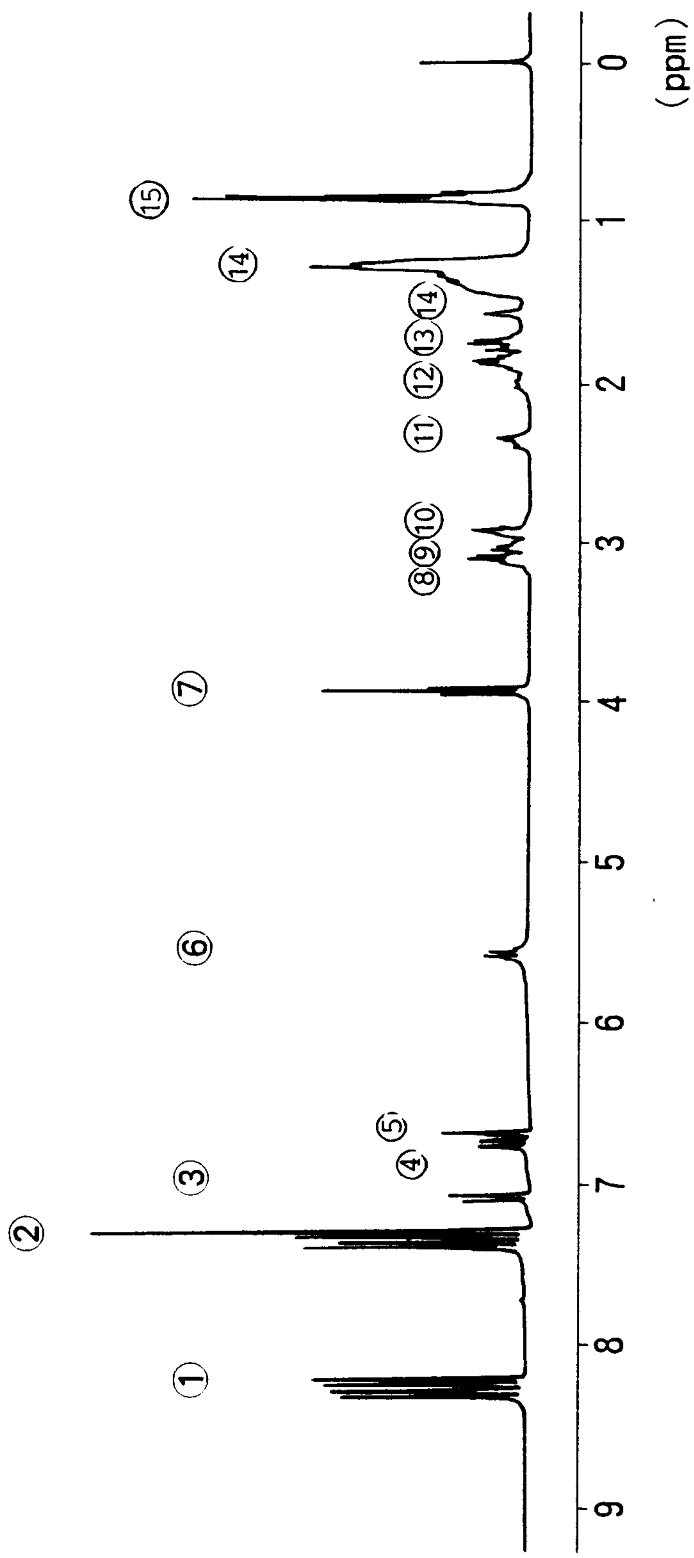

# FIG.4

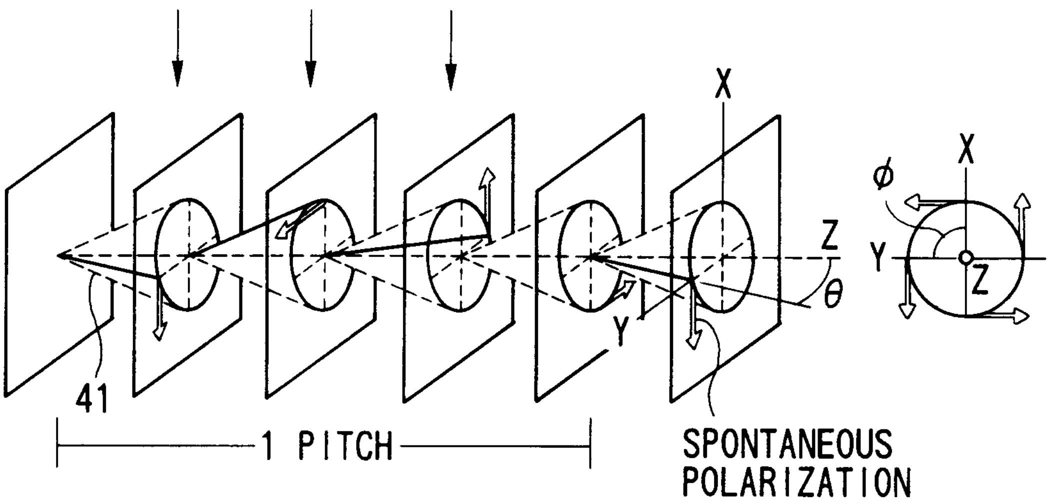


# FIG.5

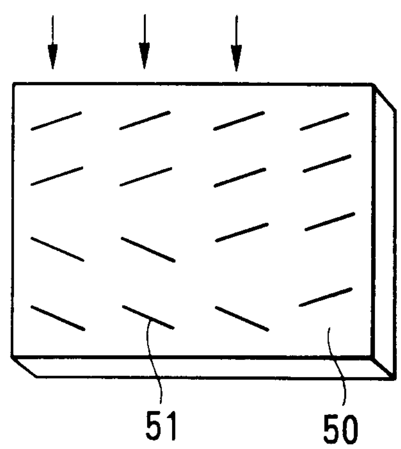

FIG.6(A)

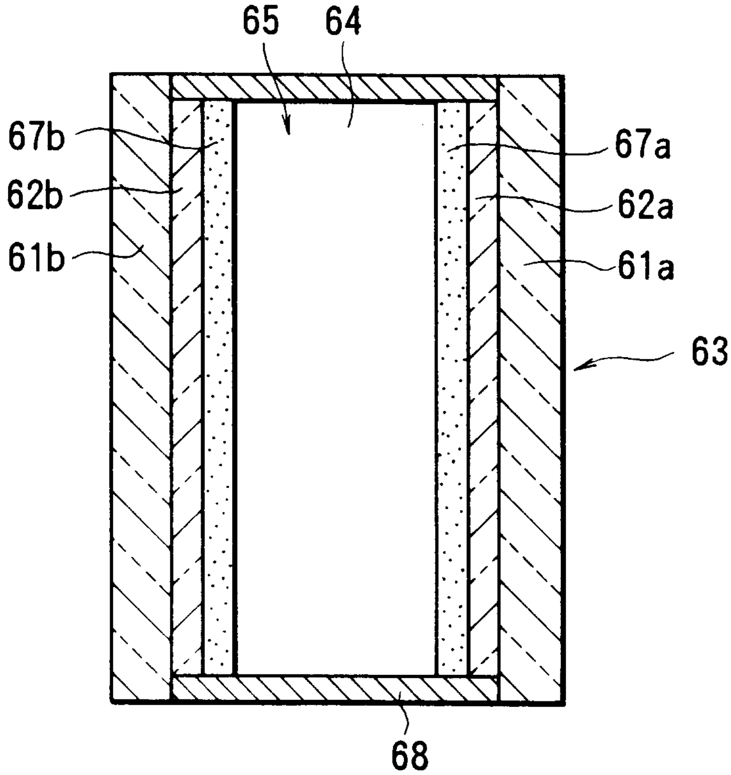
65
64
67b
62b
61b
67a
62a
61a
63
68

FIG.6(B)

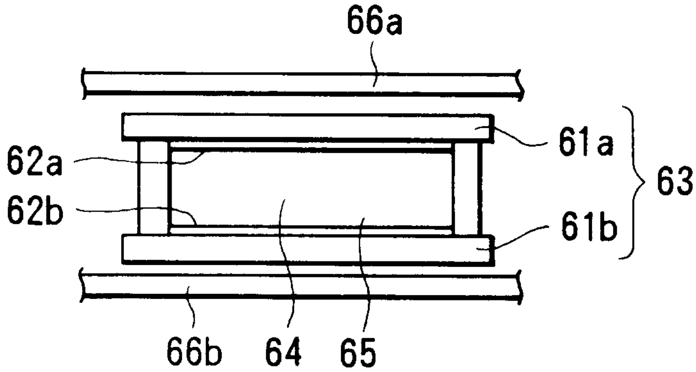
66a
62a
62b
61a
61b
63
66b
64
65

# FIG.7

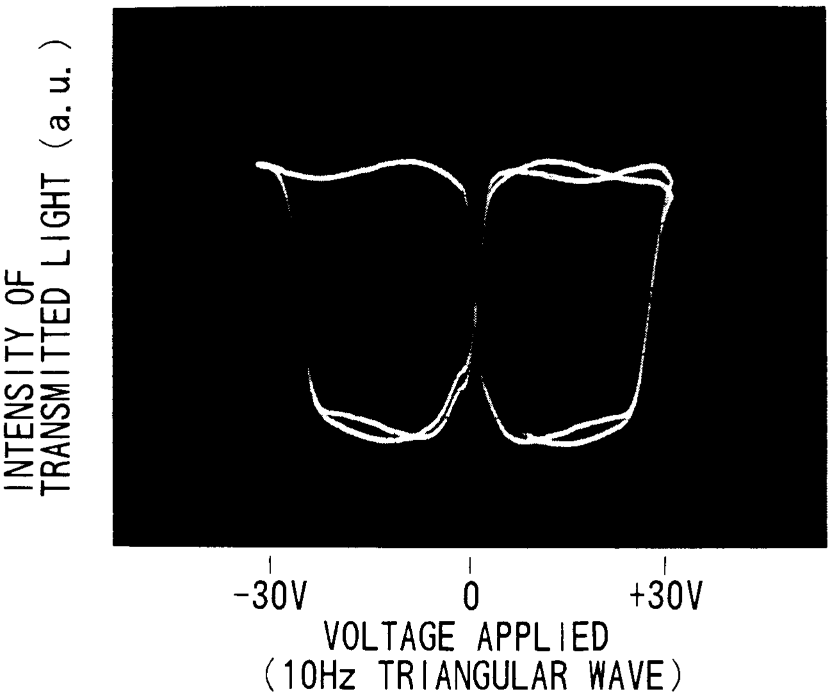
INTENSITY OF
TRANSMITTED LIGHT (a. u.)
-30V
0
+30V
VOLTAGE APPLIED
(10Hz TRIANGULAR WAVE)

# FIG.8

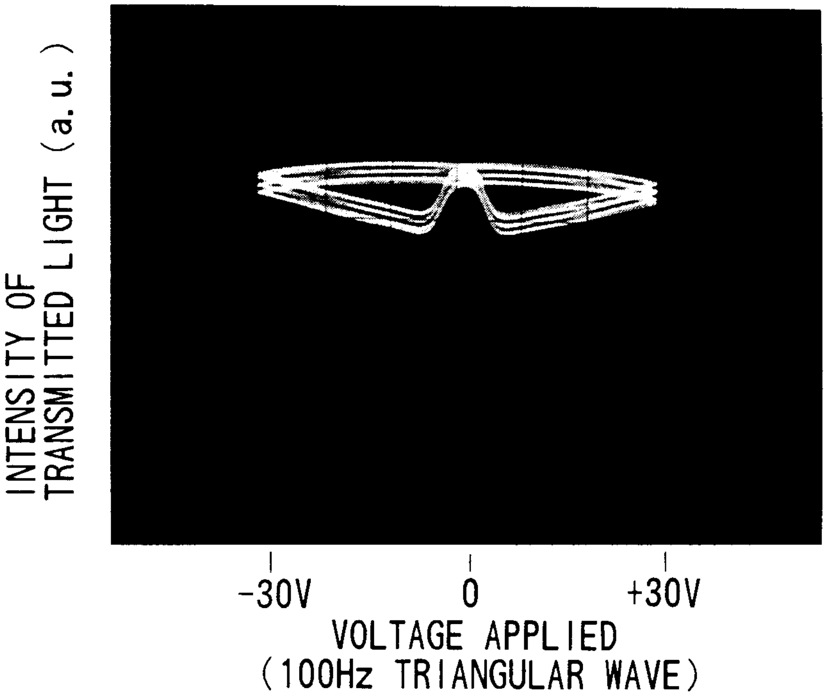
INTENSITY OF
TRANSMITTED LIGHT (a. u.)
-30V
0
+30V
VOLTAGE APPLIED
(100Hz TRIANGULAR WAVE)

FIG.9

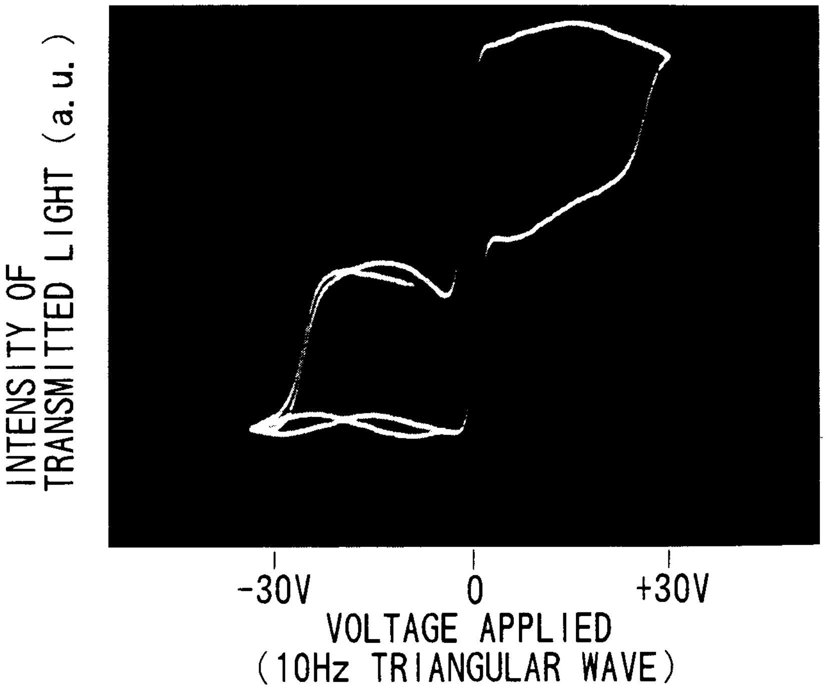
INTENSITY OF
TRANSMITTED LIGHT (a.u.)
-30V
0
+30V
VOLTAGE APPLIED
(10Hz TRIANGULAR WAVE)

FIG.10

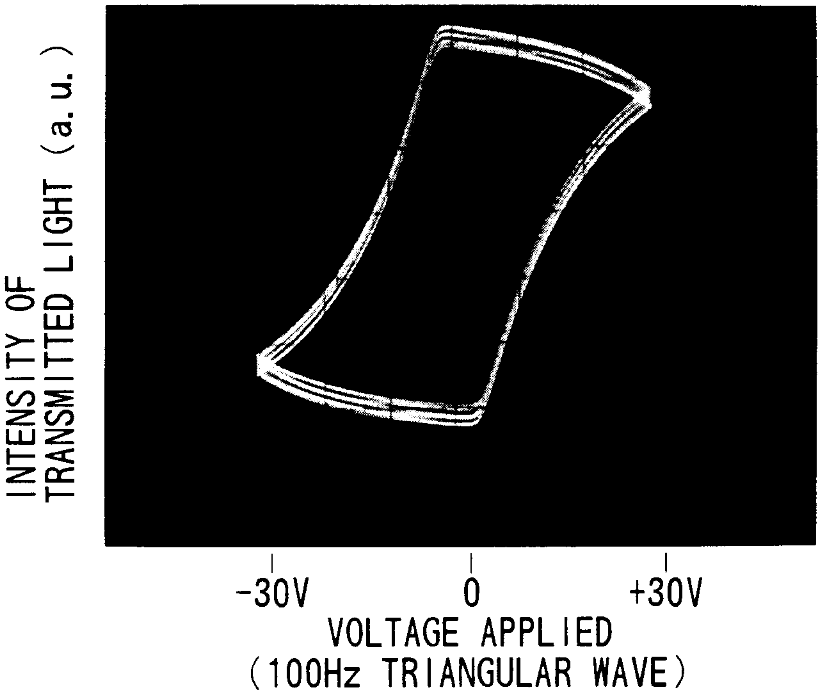
INTENSITY OF
TRANSMITTED LIGHT (a.u.)
-30V
0
+30V
VOLTAGE APPLIED
(100Hz TRIANGULAR WAVE)